# EUROPEAN PATENT SPECIFICATION

(11) **EP 3 581 587 B1**
(45) Date of publication and mention of the grant of the patent: **05.02.2025**
(21) Application number: 17895532.4
(22) Date of filing: 29.12.2017
(51) Int. Cl.: C07K 16/36, C12N 15/13, C12N 15/85, A61K 39/395, A61P 7/02, A61P 31/04

(54) **ANTI-COAGULATION FACTOR XI ANTIBODY**
ANTI-GERINNUNGSFAKTOR-XI ANTIKÖRPER
ANTICORPS ANTI-FACTEUR XI DE COAGULATION

(30) Priority: 10.02.2017 CN 201710073984
(43) Date of publication of application: 18.12.2019
(73) Proprietor: Shanghai Benemae Pharmaceutical Corporation, Shanghai 201321 (CN)
(72) Inventor: WANG, Wenyi, Shanghai 201321 (CN); YU, Quan, Shanghai 201321 (CN); LIU, Xiaowu, Shanghai 201321 (CN)
(74) Representative: Gulde & Partner
(86) International application number: PCT/CN2017/119856
(87) International publication number: WO 2018/145533

(56) References cited:
- WO-A1-2016/207858
- WO-A2-2009/067660
- WO-A2-2009/067660
- CN-A- 104 684 932
- TUCKER, E.I. ET AL.: "Prevention of Vascular Graft Occlusion and Thrombus-Associated Thrombin Generation by Inhibition of Factor XI", BLOOD, vol. 13, no. 4, 22 January 2009 (2009-01-22), pages 936 - 944, XP009116109
- MATAFONOV, A. ET AL.: "Evidence for Factor IX-Independent Roles for Factor XIa in Blood Coagulation", JOURNAL OF THROMBOSIS AND HAEMOSTASIS, vol. 11, 31 December 2013 (2013-12-31), pages 2118 - 2127, XP055293618
- SUN, Y.H. ET AL.: "Identification of a Factor IX Binding Site on the Third Apple Domain of Activated Factor XI", THE JOURNAL OF BIOLOGICAL CHEMISTRY, vol. 271, no. 46, 15 November 1996 (1996-11-15), pages 29023 - 29028, XP002435764
- LIU, HUINA ET AL.: "Coagulation Factor XI: a New Target for Antithrombotic Drug", JOURNAL OF CLINICAL AND PATHOLOGICAL RESEARCH, vol. 36, no. 6, 31 December 2016 (2016-12-31), pages 847 - 851, XP009515860
- HE, R. ET AL.: "Factor XI: Hemostasis, Thrombosis, and Antithrombosis", THROMBOSISI RESEARCH, vol. 129, 22 December 2011 (2011-12-22), pages 541 - 550, XP028914428

## Description

### TECHNICAL FIELD

This disclosure relates to antibodies capable of binding to the coagulation factor XI (FXI) and/or its activated form factor Xla (FXla), and to fragments of FXI and/or FXla, and uses thereof, including uses as anticoagulation agents for treating thrombosis that do not compromise hemostasis.

### BACKGROUND

Thrombosis is a condition that involves blood clotting in a blood vessel, thereby blocking or obstructing blood flow in the affected area. This condition can lead to serious complications if the blood clots travel along the circulatory system to a crucial body part such as heart, brain, and lungs, causing heart attack, stroke, pulmonary embolism, etc. Thrombosis can be treated or prevented by anticoagulants such as heparin and warfarin. The most common adverse effect of these currently available therapies is bleeding. Therefore, these therapies are limited by the dose and patient compliance because patients are required to be closely monitored after the treatment.
In WO 2009/067660 A2 an antibody is disclosed which binds to an epitope in domain A3 of Factor XI and its use in inhibition of thrombosis associated with sepsis.

There is a need for an effective thrombosis therapy or prophylaxis with minimal side effects. This disclosure satisfies the need in the art.

### SUMMARY

The present invention is directed to subject matter as defined in the claims.

Provided herein in certain embodiments are antibodies that bind to coagulation factor XI (FXI) and/or its activated form factor Xla (FXla), and to fragments of FXI and/or FXla as defined in the claims. In some embodiments, the antibodies are monoclonal antibodies. In some embodiments, the antibodies are recombinant antibodies. In some embodiments, the antibodies are humanized antibodies. In some embodiments, the antibodies are immunologically active portions of immunoglobulin molecules, e.g., Fabs, Fvs, or scFvs. In some embodiments, the antibodies bind to the A3 domain of FXI and/or FXla. The antibodies of the invention include CDRs consisting of or comprising the amino acid sequences of SEQ ID NOs: 123-128, as defined in the claims.

Provided herein is a pharmaceutical composition for treating and/or preventing thrombosis and/or complications or conditions associated with thrombosis. The pharmaceutical composition comprises one or more anti-FXI and/or anti-FXla antibodies of the invention. In some embodiments, the pharmaceutical composition further comprises one or more pharmaceutically acceptable adjuvants, carriers, excipients, preservatives, or a combination thereof.

Provided herein is a nucleic acid encoding an anti-FXI and/or anti-FXla antibody of the invention, or a functional fragment of either antibody, as well as a vector comprising the nucleic acid, and a host cell comprising the vector. In some embodiments, the vector is an expression vector that is capable of producing the antibody or a functional fragment thereof encoded by the nucleic acid in a host cell.

For the purpose of the present invention, any reference to a method of treatment comprising administration of the antibody of the invention or a use of the antibody of the invention for manufacturing of a medicament is to be understood as a reference to the antibody of the invention for use in such method.

Provided herein is a kit comprising one or more anti-FXI and/or anti-FXla antibodies of the invention for use in treating and/or preventing thrombosis and/or complications or conditions associated with thrombosis. Alternatively, the kit comprises a pharmaceutical composition comprising one or more anti-FXI and/or anti-FXla antibodies of the invention for use in treating and/or preventing thrombosis and/or complications or conditions associated with thrombosis. In certain embodiments, the kit further comprises instructions for use.

Provided herein is a method of treating and/or preventing thrombosis and/or complications or conditions associated with thrombosis. The method includes administering to a subject in need thereof a therapeutically effective amount of one or more anti-FXI and/or anti-FXla antibodies of the invention. Alternatively, the method includes administering to a subject in need thereof a therapeutically effective amount of a pharmaceutical composition containing an anti-FXI antibody, an anti-FXla antibody, or a functional fragment of either antibody in accordance with the invention.

Provided herein is a use of an anti-FXI and/or anti-FXla antibody of the invention in formulating a medicament for treating and/or preventing thrombosis and/or complications or conditions associated with thrombosis.

Provided herein is a method of producing an anti-FXI and/or anti-FXla antibody of the invention. The method entails the steps of transforming a host cell with a vector comprising a nucleic acid encoding the antibody of the invention, and expressing the antibody of the invention in the host cell. The method can further include purifying the expressed antibody of the invention from the host cell. Additionally, the purified antibody of the invention can be subjected to modifications such that the modified recombinant antibody retains the activity of the corresponding human antibody. Alternatively, an antibody of the invention can be produced from culturing a hybridoma.

### BRIEF DESCRIPTION OF THE DRAWINGS

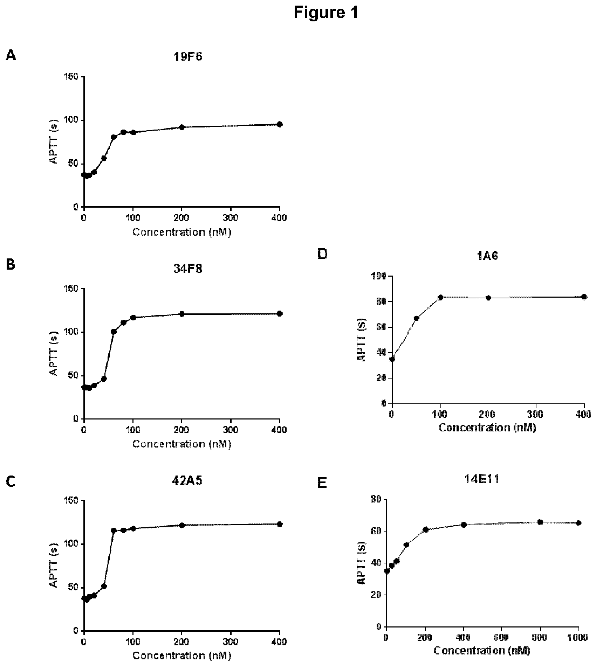

Figure 1 illustrates the effects of five anti-FXI antibodies via APTT assay in human plasma. Human plasma supplemented with five different antibodies at a concentration ranging from 0 to 400 nM were tested in an APTT assay as described in Example 2. The five antibodies tested included 19F6 (A), 34F8 (B), 42A5 (C), 1A6 (D), and 14E11 (E). Antibodies 1A6 and 14E11 were used as positive controls in this experiment.

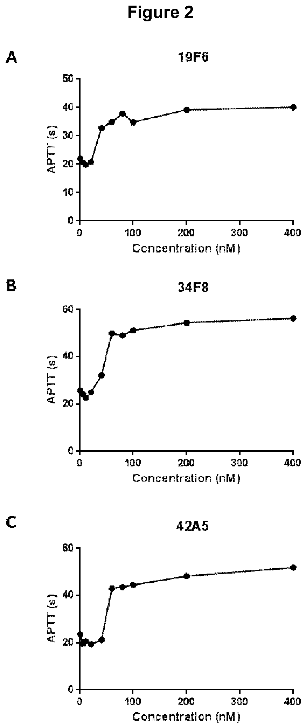

Figure 2 illustrates the effects of antibodies 19F6 (A), 34F8 (B), and 42A5 (C) on the APTT assay in monkey plasma. Monkey plasma supplemented with three different antibodies at a concentration ranging from 0 to 400 nM were tested in an APTT assay as described in Example 3.

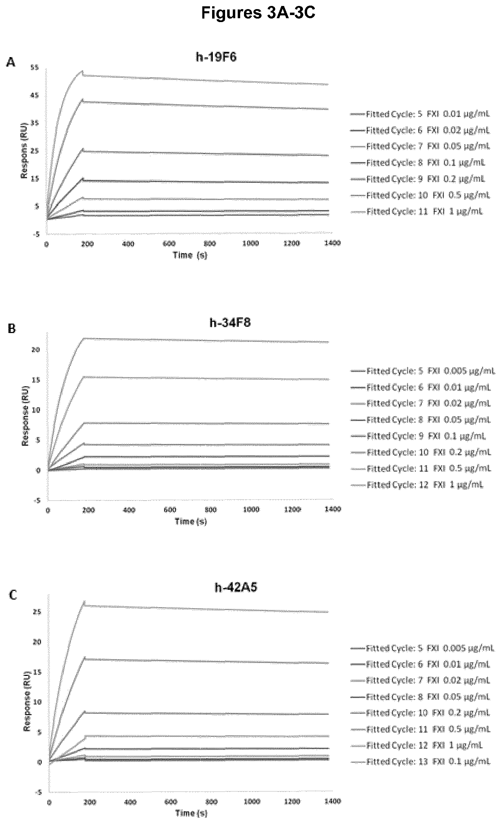

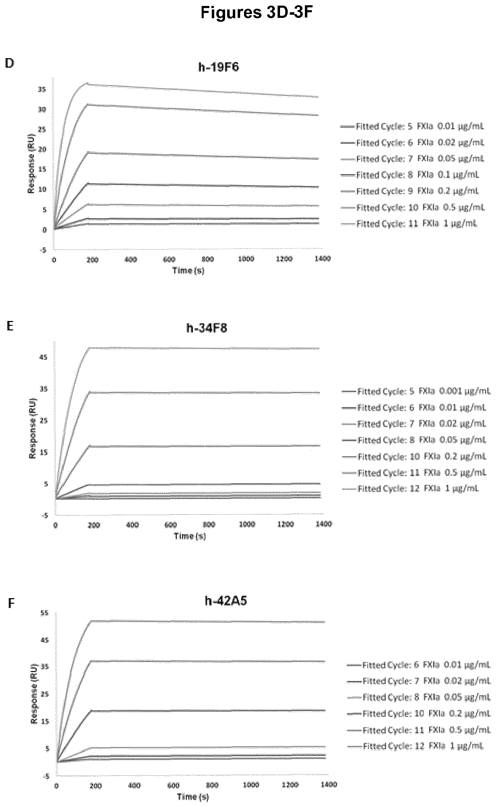

Figure 3 illustrates SPR sensorgrams for FXI binding to immobilized h-19F6 (A), h-34F8 (B), and h-42A5 (C), as well as SPR sensorgrams for FXla binding to immobilized h-19F6 (D), h-34F8 (E), and h-42A5 (F). Data were fit with 1:1 binding model, and curve fits at test concentrations of FXI (0.005 - 1 ng/mL) are shown overlaid on the sensorgrams. Each curve indicates a different test concentration of FXI or FXla.

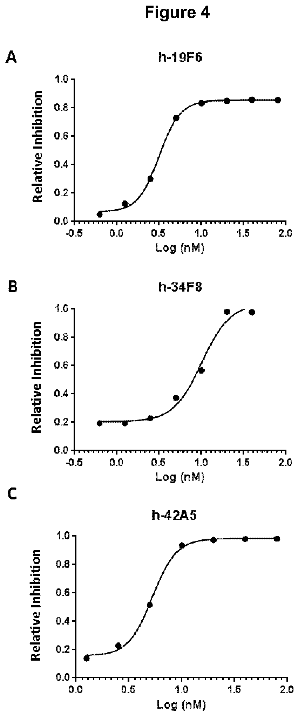

Figure 4 illustrates the concentration-response curves of antibodies h-19F6 (A), h-34F8 (B), and h-42A5 (C) inhibiting human FXla from hydrolyzing S-2366.

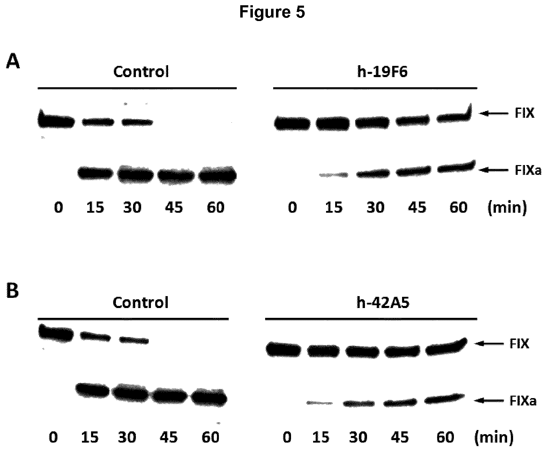

Figure 5 illustrates the inhibitory effects of antibodies h-19F6 (A) and h-42A5 (B) on FXla-mediated activation of FIX to FIXa. Human FIX (200 nM) was incubated with FXla (5 nM) in PBS with 5 mM CaCl₂ at room temperature with 1 µM control IgG or h-19F6 or h-42A5. At the indicated intervals, samples were collected and the FIX as well as FIXa was determined by Western blots using goat anti-human FIX IgG (Affinity Biologicals).

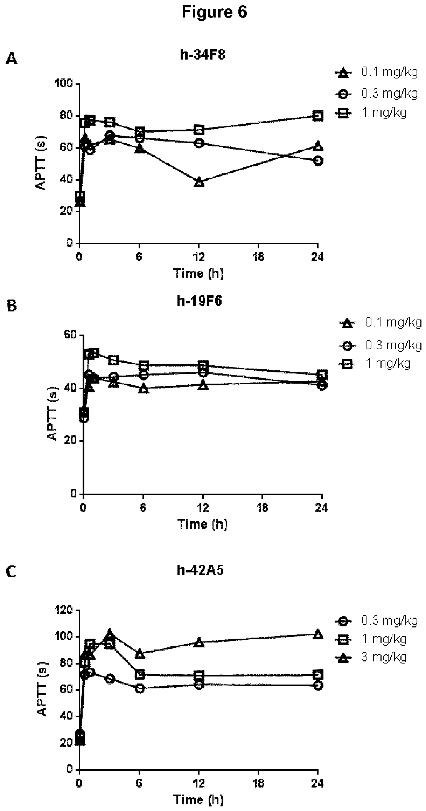

Figure 6 illustrates the effects of antibodies h-34F8, h-19F6, and h-42A5 on APTT in cynomolgus monkeys. Monkeys were intravenously administered with indicated doses of h-34F8 (A), h-19F6 (B), and h-42A5 (C). *Ex vivo* clotting time APTT was determined at pre-dose (time 0), and 0.5, 1, 3, 6, 12, and 24 hours post-dose.

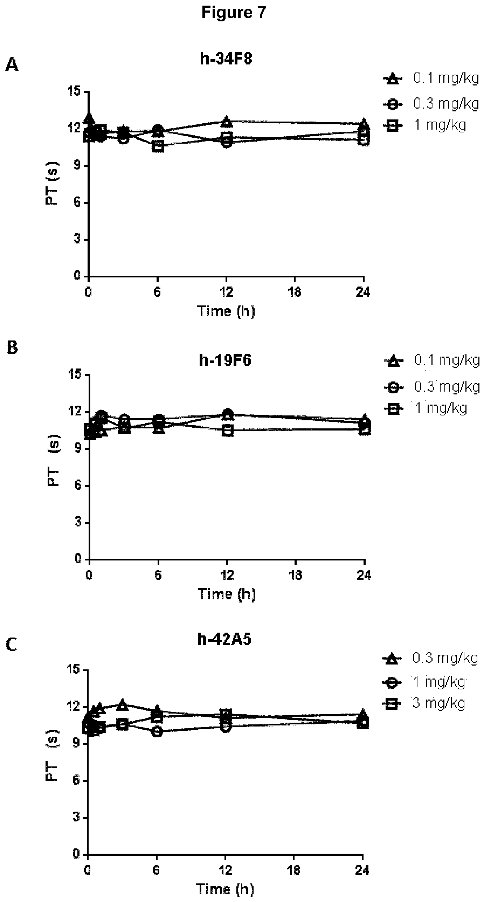

Figure 7 illustrates the effects of antibodies h-34F8, h-19F6, and h-42A5 on PT in cynomolgus monkeys. Monkeys were intravenously administered with the indicated doses of h-34F8 (A), h-19F6 (B), and h-42A5 (C). *Ex vivo* clotting time PT was determined at pre-dose (time 0), and 0.5, 1, 3, 6, 12, and 24 hours post-dose.

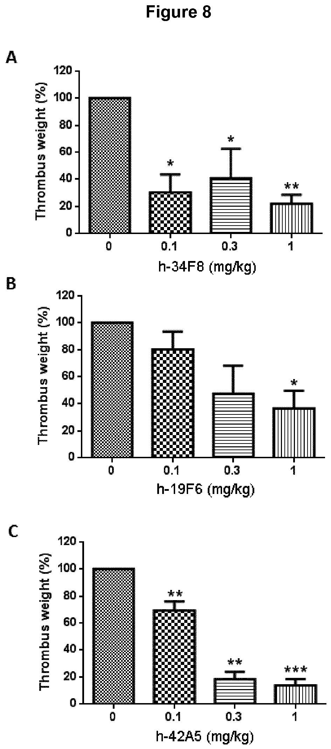

Figure 8 illustrates the effects of antibodies h-34F8, h-19F6, and h-42A5 on AV shunt thrombosis in cynomolgus monkeys. Escalating levels of h-34F8 (A), h-19F6 (B), or h-42A5 (C) were intravenously administered to monkeys (n=3 for h-34F8 and h-19F6; n=4 for h-42A5), changes of clot weight from pre-dose were determined in monkey model of AV shunt thrombosis. **P* < 0.05, ***P* < 0.01 and ****P* < 0.001 vs. Vehicle.

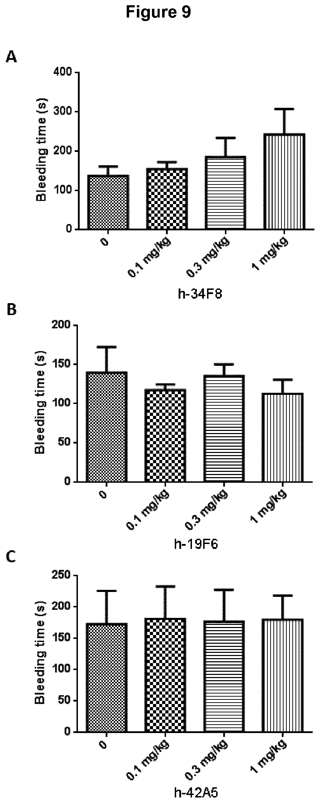

Figure 9 illustrates the effects of antibodies h-34F8, h-19F6, and h-42A5 on bleeding time in cynomolgus monkeys. Escalating levels of h-34F8 (A), h-19F6 (B), or h-42A5 (C) were intravenously administered to monkeys (n=3 for 34F8 and h-19F6, n=4 for h-42A5), bleeding time was assessed at pre-dose and at 30 min post each dose.

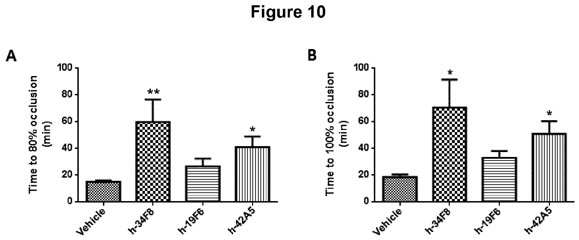

Figure 10 illustrates the antithrombotic effects of antibodies h-34F8, h-19F6, and h-42A5. Four groups of monkeys (n = 5) were intravenously administered with the vehicle, 0.3 mg/kg of h-34F8, h-19F6, or h-42A5, for 2 hours, and FeCl₃ was applied on the left femoral artery of each animal to induce thrombosis. The time to 80% thrombotic occlusion (A) and to 100% thrombotic occlusion (B) were determined by monitoring the blood flow velocity. **P* < 0.05 and ***P* < 0.01 vs. vehicle.

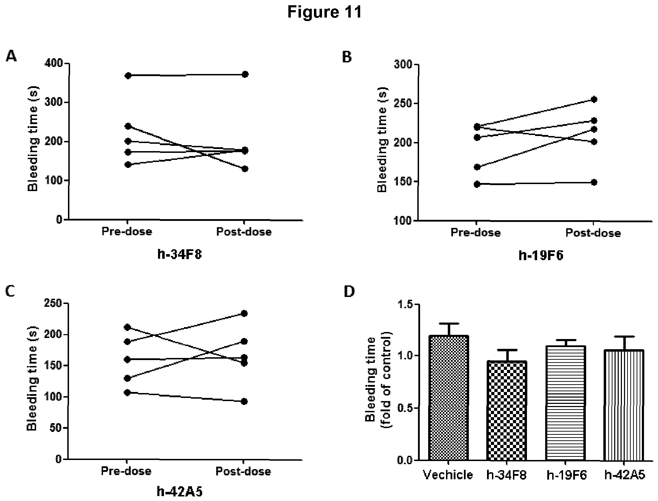

Figure 11 illustrates that the treatment with antibodies h-34F8, h-19F6, or h-42A5 did not prolong the bleeding time in monkeys. Four groups of monkeys (n = 5) were intravenously administered with the vehicle, 0.3 mg/kg of h-34F8, h-19F6, or h-42A5, and template bleeding time was measured pre-dose and 1 hour post-dose. The individual bleeding time in h-34F8, h-19F6, and h-42A5 treated group is shown in (A), (B) and (C), respectively. The bleed time change upon vehicle, h-34F8, h-19F6, or h-42A5 treatment is shown in (D).

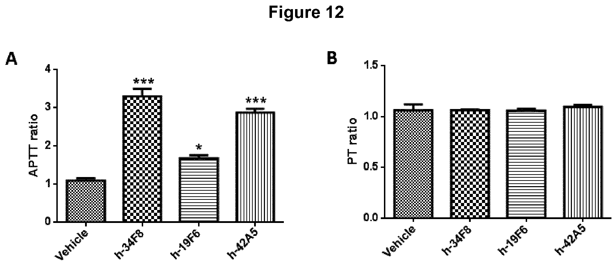

Figure 12 illustrates the effects of antibodies h-34F8, h-19F6, and h-42A5 on clotting times of monkey plasma. Four groups of monkeys (n = 5) were intravenously administered with the vehicle, 0.3 mg/kg of h-34F8, h-19F6, and h-42A5, respectively, and blood was collected pre-dose and about 3 hours post-dose for plasma preparation and clotting time APTT and PT determination. The APTT changes and PT changes are shown in (A) and (B), respectively. ***P* < 0.01 and ****P* < 0.001 vs. vehicle.

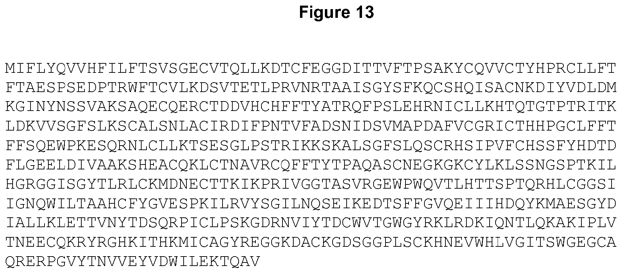

Figure 13 illustrates the amino acid sequence of human FXI (SEQ ID NO: 203).

### DETAILED DESCRIPTION

The present invention is as defined in the claims.

Both intrinsic pathway and extrinsic pathway are involved in *in vivo* blood coagulation cascades. The intrinsic pathway, also called the contact activation pathway, is initiated by contact with a surface interface and results in activation of FXII. The intrinsic pathway also involves FXI, FIX and FVIII. The extrinsic pathway, also called the tissue factor (TF) pathway, is initiated by vascular injury and results in the formation of an activated complex of TF-FVlla. These two pathways meet and activate the common pathway, leading to conversion of prothrombin to thrombin and eventually the formation of cross-linked fibrin clot. The antibodies disclosed herein binds to FXI and/or FXla and target the intrinsic pathway of blood coagulation. The structure of FXI and FXI's involvement in blood coagulation have been reported in various publications. See, for example, Emsley et al., Blood 115(13): 2569-2577 (2010).

### Anti-FXI or Anti-FXIa Antibodies

Provided herein are antibodies that bind to FXI, FXla, and/or a fragment of FXI or FXla and inhibit the formation of blood clot. These antibodies are capable of binding to FXI, FXla, and/or a fragment of FXI or FXla (e.g., a fragment comprising the A3 domain) and exhibiting an inhibitory effect at a concentration that is much lower than the maximum safety dose. For example, in some embodiments a dose of the antibody between 0.1 mg/kg i.v. and 3 mg/kg i.v. exhibits an inhibitory effect on conversion of FXI to FXla in cynomolgus monkeys. Moreover, the antibodies disclosed herein can be used as anticoagulation agents with superior safety due to their minimal risk of causing bleeding versus conventional anticoagulation agents such as heparin.

As used herein, the term "comprising" with regard to a composition or method means that the composition or method includes at least the recited elements. The term "consisting essentially of" means that the composition or method includes the recited elements, and may further include one or more additional elements that do not materially affect the novel and basic characteristics of the composition or method. For example, a composition consisting essentially of recited elements may include those recited elements plus one or more trace contaminants from the isolation and purification method, pharmaceutically acceptable carriers such as phosphate buffered saline, preservatives, and the like. The term "consisting of" means the composition or method includes only the recited elements. Embodiments defined by each of the transitional terms are within the scope of this invention.

The term "antibody" as used herein refers to an immunoglobulin molecule or an immunologically active portion thereof that specifically binds to, or is immunologically reactive with a particular antigen, for example, FXI, FXla, or a particular domain or fragment of FXI or FXla, e.g., the A3 domain. In certain embodiments an antibody for use in the present methods, compositions, and kits is a full-length immunoglobulin molecule, which comprises two heavy chains and two light chains, with each heavy and light chain containing three complementary determining regions (CDRs). The term "antibody," in addition to natural antibodies, also includes genetically engineered or otherwise modified forms of immunoglobulins, such as synthetic antibodies, intrabodies, chimeric antibodies, fully human antibodies, humanized antibodies, peptibodies and heteroconjugate antibodies (e.g., bispecific antibodies, multispecific antibodies, dualspecific antibodies, anti-idiotypic antibodies, diabodies, triabodies, and tetrabodies). The antibodies disclosed herein can be monoclonal antibodies or polyclonal antibodies. In those embodiments, wherein an antibody is an immunologically active portion of an immunoglobulin molecule, the antibody may be, for example, a Fab, Fab', Fv, Fab' F(ab')₂, disulfide-linked Fv, single chain Fv antibody (scFv), single domain antibody (dAb), or diabody. The antibodies disclosed herein, including those that are immunologically active portion of an immunoglobulin molecule, retain the ability to bind a specific antigen, for example FXI or FXla, or to bind a specific fragment of FXI or FXla such as the A3 domain.

In some embodiments, the anti-FXI and/or anti-FXla antibodies disclosed herein have undergone post-translational modifications such as phosphorylation, methylation, acetylation, ubiquitination, nitrosylation, glycosylation, or lipidation associated with expression in a mammalian cell line, including a human or a non-human host cell. Techniques for producing recombinant antibodies and for *in vitro* and *in vivo* modifications of recombinant antibodies are known in the art. See, e.g., Liu et al., mAbs 6(5): 1145-1154 (2014).

Also disclosed are polynucleotides or nucleic acids encoding the anti-FXI and/or anti-FXla antibodies disclosed herein. In some embodiments, the polynucleotide or nucleic acid includes DNA, mRNA, cDNA, plasmid DNA. The nucleic acid encoding the antibody or a functional fragment thereof disclosed herein can be cloned into a vector, such as a pTT5 mammalian expression vector, which may further include a promoter and/or other transcriptional or translational control elements such that the nucleic acid can be expressed to produce the antibody or the functional fragment thereof.

The nucleic acid (DNA) and/or amino acid (PRT) sequences, including the sequences of the VH and VL and CDRs, of some examples of the antibodies disclosed herein are listed in Table 1 below, wherein the notifier 34F8 refers to an antibody according to the present invention and the notifiers 3G12, 5B2, 7C9, 7F1, 13F4, 19F6, 21F12, 38E4, 42A5, 42F4, 45H1, 14E11 and 1A6 refer to antibodies not in accordance with the present invention.

**Table 1: Antibody Sequences**

| **Description** | **SEQ ID NO** | **Type** | **Sequence** |
|---|---|---|---|
| 3G12-VL | 1 | DNA | GACATTGTGCTGACCCAATCTCCAGCTTCTTTGGC TGTGTCTCTAGGGCAGAGGGCCACCATCTCCTGC AGAGCCAGCGAAAGTGTTGATAATTATGCCATTAG TTTTATGAACTGGTTCCAACAGAAACCAGGACAGC CACCCAAACTCCTCATCTATGCTGCATCCAACCTA GGATCCGGGGTCCCTGCCAGGTTTAGTGGCAGTG GGTCTGGGACAGACTTCAGCCTCAACATCCATCCT ATGGAGGAGGATGATACTGCAATGTATTTCTGTCA GCAAGATAAGGAGGTTCCGTGGACGTTCGGTGGA GGCACCGAGCTGGAAATCAAA |
| 3G12-VH | 2 | DNA | CAGGTCACTCTGAAAGAGTCTGGCCCTGGGATATT GCAGCCCTCCCAGACCCTCAGTCTGACTTGTTCTT TCTCTGGGTTTTCACTGAACACTCCTGGTATGGGT GTGAGCTGGATTCGTCAGCCTTCAGGAAAGGGTC TGGAATGGCTGGCACACATTTACTGGGATGATGAC AAGCGCTTTAACCCATCCCTGAAGAGCCGACTCAC AATCTCCAAGGATACCTCCAGAGATCAGGTATTCC TCATGATCACCAGTGTGGACACTGCAGATTCTGCC ACATACTTCTGTGCTCGAAAAGGCCGCGGGCCCT TTACTTACTGGGGCCAAGGGACTCTGGTCACTGTC TCTTCA |
| 3G12-CDR-L1 | 3 | DNA | AGAGCCAGCGAAAGTGTTGATAATTATGCCATTAG TTTTATGAAC |
| 3G12-CDR-L2 | 4 | DNA | GCTGCATCCAACCTAGGATCC |
| 3G12-CDR-L3 | 5 | DNA | CAGCAAGATAAGGAGGTTCCGTGGACG |
| 3G12-CDR-H1 | 6 | DNA | ACTCCTGGTATGGGTGTGAGC |
| 3G12-CDR-H2 | 7 | DNA | CACATTTACTGGGATGATGACAAGCGCTTTAACCC ATCCCTGAAGAGC |
| 3G12-CDR-H3 | 8 | DNA | AAAGGCCGCGGGCCCTTTACTTAC |
| 3G12-VL | 9 | PRT | DIVLTQSPASLAVSLGQRATISCRASESVDNYAISFM NWFQQKPGQPPKLLIYAASNLGSGVPARFSGSGSG TDFSLNIHPMEEDDTAMYFCQQDKEVPWTFGGGTE LEIK |
| 3G12-VH | 10 | PRT | QVTLKESGPGILQPSQTLSLTCSFSGFSLNTPGMGV SWIRQPSGKGLEWLAHIYWDDDKRFNPSLKSRLTIS KDTSRDQVFLMITSVDTADSATYFCARKGRGPFTYW GQGTLVTVSS |
| 3G12-CDR-L1 | 11 | PRT | RASESVDNYAISFMN |
| 3G12-CDR-L2 | 12 | PRT | AASNLGS |
| 3G12-CDR-L3 | 13 | PRT | QQDKEVPWT |
| 3G12-CDR-H1 | 14 | PRT | TPGMGVS |
| 3G12-CDR-H2 | 15 | PRT | HIYWDDDKRFNPSLKS |
| 3G12-CDR-H3 | 16 | PRT | KGRGPFTY |
| 5B2-VL | 17 | DNA | GACATTGTGCTGACCCAATCTCCAGCCTCTTTGGC TGTGTCTCTAGGGCAGAGGGCCACCATCTCCTGC AGAGCCAGCGAAAGTGTTGATAATTATGGCATTAG TTTTCTGAACTGGTTCCAACAGAAACCAGGACAGC CACCCAAACTCCTCATCTATGCTGCATCCAATCTA GGATCCGGGGTCCCTGCCAGGTTTAGTGGCAGTG GGTCTGGGACAGACTTCAGCCTCAACATCCATCCT ATGGAGGAGGATGATACTGCAATGTATTTCTGTCA GCAAGATAAGGGGGTTCCGTGGACGTTCGGTGGA GGCACCAAGCTGGAAATGAAA |
| 5B2-VH | 18 | DNA | CAGGTTACTCTGAAAGAGTCTGGCCCTGGGATATT GCAGCCCTCCCAGACCCTCAGTCTGACTTGTTCTT TCTCTGGGTTTTCACTGAACACTTCTGGTATGGGT GTGAGCTGGATTCGTCAGCCTTCAGGAAAGGGTC TGGAGTGGCTGGCACACATTTACTGGGATGATGA CAAGCGCTATAAACCATCCCTGAAGAGCCGGCTC ACAATCTCCAAGGATACCTCCAGAAACCAGGTATT CCTCATGATCACCAGTGTGGACACTGCAGATACTG CCACATACTACTGTGTTCGAAAAGGCCGCGGCC CTTTGCTAACTGGGGCCAAGGGACTCTGGTCACT GTCTCTGCA |
| 5B2-CDR-L1 | 19 | DNA | AGAGCCAGCGAAAGTGTTGATAATTATGGCATTAG TTTTCTGAAC |
| 5B2-CDR-L2 | 20 | DNA | GCTGCATCCAATCTAGGATCC |
| 5B2-CDR-L3 | 21 | DNA | CAGCAAGATAAGGGGGTTCCGTGGACG |
| 5B2-CDR-H1 | 22 | DNA | ACTTCTGGTATGGGTGTGAGC |
| 5B2-CDR-H2 | 23 | DNA | CACATTTACTGGGATGATGACAAGCGCTATAAACC ATCCCTGAAGAGC |
| 5B2-CDR-H3 | 24 | DNA | AAAGGCCGCGGGCCCTTTGCTAAC |
| 5B2-VL | 25 | PRT | DIVLTQSPASLAVSLGQRATISCRASESVDNYGISFLN WFQQKPGQPPKLLIYAASNLGSGVPARFSGSGSGT DFSLNIHPMEEDDTAMYFCQQDKGVPWTFGGGTKL EMK |
| 5B2-VH | 26 | PRT | QVTLKESGPGILQPSQTLSLTCSFSGFSLNTSGMGV SWIRQPSGKGLEWLAHIYWDDDKRYKPSLKSRLTIS KDTSRNQVFLMITSVDTADTATYYCVRKGRGPFANW GQGTLVTVSA |
| 5B2-CDR-L1 | 27 | PRT | RASESVDNYGISFLN |
| 5B2-CDR-L2 | 28 | PRT | AASNLGS |
| 5B2-CDR-L3 | 29 | PRT | QQDKGVPWT |
| 5B2-CDR-H1 | 30 | PRT | TSGMGVS |
| 5B2-CDR-H2 | 31 | PRT | HIYWDDDKRYKPSLKS |
| 5B2-CDR-H3 | 32 | PRT | KGRGPFAN |
| 7C9-VL | 33 | DNA | GACATCCAGATGACCCAGTCTCCATCCTCCTTATC TGCCTCTCTGGGAGAAAGAGTCAGTCTCACTTGTC GGGCAAGTCAGGACATTGATATTCGCTTAAACTGG CTTCGACAGGAACCAGATGGAACTATTAAACGCCT GATCTACGCCACATCCAGTTTAGATTCTGGTGTCC CCAAAAGGTTCAGTGGCAGTAGGTCTGGGTCAGA TTATTCTCTCACCATCAGCAGCCTTGAGTCTGAAG ATTTTGTTGACTATTACTGTCTACAATATGCTAGTT CTCCATTCACGTTCGGCTCGGGGACAAAGTTGGA AATAAAA |
| 7C9-VH | 34 | DNA | CAGATCCAGTTGGTGCAGTCTGGACCTGAACTGA AGAAGCCTGGAGAGACCGTCAAGATCTCCTGCAA GGCTTCTGGGTATATTTTCACAGACTATGGAATGA ACTGGGTGAAGCAGGCTCCAGGAAAGGGTTTAAA GTGGATGGGCTGGATAAACACCTACACTGGAGAG CCAACATATGCTGATGACTTCAAGGGACGGTTTGT CTTCTCTTTGGAAACCTCTGCCAGCACTGCCTATT TACAGATCAACAACCTCAAAAATGAGGACACGGCT ACATTTTTCTGTGCAAGAAGGAGGATGGGTTATGC TGTGGACTACTGGGGTCAAGGAACCTCAGTCACC GTCTCCTCA |
| 7C9-CDR-L1 | 35 | DNA | CGGGCAAGTCAGGACATTGATATTCGCTTAAAC |
| 7C9-CDR-L2 | 36 | DNA | GCCACATCCAGTTTAGATTCT |
| 7C9-CDR-L3 | 37 | DNA | CTACAATATGCTAGTTCTCCATTCACG |
| 7C9-CDR-H1 | 38 | DNA | GACTATGGAATGAAC |
| 7C9-CDR-H2 | 39 | DNA | TGGATAAACACCTACACTGGAGAGCCAACATATGC TGATGACTTCAAGGGA |
| C9-CDR-H3 | 40 | DNA | AGGAGGATGGGTTATGCTGTGGACTAC |
| 7C9-VL | 41 | PRT | DIQMTQSPSSLSASLGERVSLTCRASQDIDIRLNWLR QEPDGTIKRLIYATSSLDSGVPKRFSGSRSGSDYSLT ISSLESEDFVDYYCLQYASSPFTFGSGTKLEIK |
| 7C9-VH | 42 | PRT | QIQLVQSGPELKKPGETVKISCKASGYIFTDYGMNW VKQAPGKGLKWMGWINTYTGEPTYADDFKGRFVFS LETSASTAYLQINNLKNEDTATFFCARRRMGYAVDY WGQGTSVTVSS |
| 7C9-CDR-L1 | 43 | PRT | RASQDIDIRLN |
| 7C9-CDR-L2 | 44 | PRT | ATSSLDS |
| 7C9-CDR-L3 | 45 | PRT | LQYASSPFT |
| 7C9-CDR-H1 | 46 | PRT | DYGMN |
| 7C9-CDR-H2 | 47 | PRT | WINTYTGEPTYADDFKG |
| 7C9-CDR-H3 | 48 | PRT | RRMGYAVDY |
| 7F1-VL | 49 | DNA | GACATTGTGCTGACCCAATCTCCAGCTTCTTTGGC TGTGTCTCTAGGGCAGAGGGCCACCATCTCCTGC AGAGCCAGCGAAAGTGTTGATAATTATGCCATTAG TTTTATGAATTGGTTCCAACAGAAACCAGGACAGC CACCCAAACTCCTCATCTATGCTGCATCCAACCTA GGATCCGGGGTCCCTGCCAGGTTTAGTGGCAGTG GGTCTGGGACAGACTTCAGCCTCAACATCCATCCT ATGGAGGAGGATGATACTGCAATGTATTTCTGTCA GCAAGATAAGGAGGTTCCGTGGACGTTCGGTGGA GGCACCAAGCTGGAGCTGAAA |
| 7F1-VH | 50 | DNA | CAGGTTACTCTGAAAGAGTCTGGCCCTGGGATAG TGCAGCCCTCCCAGACCCTCAATCTGACTTGTTCT TTCTCTGGATTTTCACTGAGCACTTCTGGTATGGG TGTGAGCTGGATTCGTCAGCCTTCAGGAAAGGGT CTGGATTGGCTGGCACACATTTACTGGGATGATGA CAAGCGCTATAACCCATCCCTGATGAGCCGGCTC ACAATCTCCAAGGATACCTCCAGAAACCAGGTATT CCTCATGATCACCAGTGTGGACACTGCAGATACTG CCACATACTACTGTGCTCGAAAAGGCCGCGGCC CTTTGCTTACTGGGGCCAAGGGACTCTGGTCACT GTCTCTTCA |
| 7F1-CDR-L1 | 51 | DNA | AGAGCCAGCGAAAGTGTTGATAATTATGCCATTAG TTTTATGAAT |
| 7F1-CDR-L2 | 52 | DNA | GCTGCATCCAACCTAGGATCC |
| 7F1-CDR-L3 | 53 | DNA | CAGCAAGATAAGGAGGTTCCGTGGACG |
| 7F1-CDR-H1 | 54 | DNA | ACTTCTGGTATGGGTGTGAGC |
| 7F1-CDR-H2 | 55 | DNA | CACATTTACTGGGATGATGACAAGCGCTATAACCC ATCCCTGATGAGC |
| 7F1-CDR-H3 | 56 | DNA | AAAGGCCGCGGGCCCTTTGCTTAC |
| 7F1-VL | 57 | PRT | DIVLTQSPASLAVSLGQRATISCRASESVDNYAISFM NWFQQKPGQPPKLLIYAASNLGSGVPARFSGSGSG TDFSLNIHPMEEDDTAMYFCQQDKEVPWTFGGGTK LELK |
| 7F1-VH | 58 | PRT | QVTLKESGPGIVQPSQTLNLTCSFSGFSLSTSGMGV SWIRQPSGKGLDWLAHIYWDDDKRYNPSLMSRLTIS KDTSRNQVFLMITSVDTADTATYYCARKGRGPFAYW GQGTLVTVSS |
| 7F1-CDR-L1 | 59 | PRT | RASESVDNYAISFMN |
| 7F1-CDR-L2 | 60 | PRT | AASNLGS |
| 7F1-CDR-L3 | 61 | PRT | QQDKEVPWT |
| 7F1-CDR-H1 | 62 | PRT | TSGMGVS |
| 7F1-CDR-H2 | 63 | PRT | HIYWDDDKRYNPSLMS |
| 7F1-CDR-H3 | 64 | PRT | KGRGPFAY |
| 13F4-VL | 65 | DNA | CAGTTCACGCTGACTCAACCAAAGTCCGTGTCAG GATCTTTAAGAAGCACTATCACCATTCCCTGTGAG CGCAGCAGTGGTGACATTGGAGATAGCTATGTGA GCTGGTACCAACAACACTTGGGAAGACCCCCCAT CAATGTGATCTATGCTGATGATCAAAGACCATCTG AAGTGTCTGCTCGGTTCTCGGGCTCCATCGACAG CTCCTCTAACTCAGCCTCACTGACCATCACTAATC TACAGATGGATGATGAGGCCGACTACTTCTGTCAG TCTTACGATACTTATATGGATGTTGTGTTCGGTGG TGGAACCAAGCTCAATGTCCTA |
| 13F4-VH | 66 | DNA | GAGGTGCAGCTGAAGGAATCAGGACCTGGTCTGG TGCAGCCCTCACAGACCCTGTCCCTCACCTGCAC TGTCTCTGGATTCTCATTAACGGACTACAGTGTAC ACTGGGTTCGCCAGCCTCCAGGAAAAGGTCTGGA GTGGATGGGAGTAATGTGGAGTGGTGGAAGCACA GCATATAATCCAGCTCTCACATCCCGACTGACCAT TAGCAGGGACACCTCCAAGAGCCAAGTTTTCTTAA AAATGAACAGTCTGCAAACTGAAGATACAGCCATT TACTACTGTACCAGAGCACCTTTTAACAACTGGGG CAATTGGCTTCCTTACTGGGGCCAAGGCACTCTG GTCACTGTCTCTTCA |
| 13F4-CDR-L1 | 67 | DNA | GAGCGCAGCAGTGGTGACATTGGAGATAGCTATG TGAGC |
| 13F4-CDR-L2 | 68 | DNA | GCTGATGATCAAAGACCATCT |
| 13F4-CDR-L3 | 69 | DNA | CAGTCTTACGATACTTATATGGATGTTGTG |
| 13F4-CDR-H1 | 70 | DNA | GACTACAGTGTACAC |
| 13F4-CDR-H2 | 71 | DNA | GTAATGTGGAGTGGTGGAAGCACAGCATATAATCC AGCTCTCACATCC |
| 13F4-CDR-H3 | 72 | DNA | GCACCTTTTAACAACTGGGGCAATTGGCTTCCTTA C |
| 13F4-VL | 73 | PRT | QFTLTQPKSVSGSLRSTITIPCERSSGDIGDSYVSWY QQHLGRPPINVIYADDQRPSEVSARFSGSIDSSSNSA SLTITNLQMDDEADYFCQSYDTYMDVVFGGGTKLNV L |
| 13F4-VH | 74 | PRT | EVQLKESGPGLVQPSQTLSLTCTVSGFSLTDYSVHW VRQPPGKGLEWMGVMWSGGSTAYNPALTSRLTISR DTSKSQVFLKMNSLQTEDTAIYYCTRAPFNNWGNW LPYWGQGTLVTVSS |
| 13F4-CDR-L1 | 75 | PRT | ERSSGDIGDSYVS |
| 13F4-CDR-L2 | 76 | PRT | ADDQRPS |
| 13F4-CDR-L3 | 77 | PRT | QSYDTYMDVV |
| 13F4-CDR-H1 | 78 | PRT | DYSVH |
| 13F4-CDR-H2 | 79 | PRT | VMWSGGSTAYNPALTS |
| 13F4-CDR-H3 | 80 | PRT | APFNNWGNWLPY |
| 19F6-VL | 81 | DNA | CAATTCACGCTGACTCAACCAAAGTCCGTGTCAGG CTCTTTAAGAAGCACTATCACCATTCCCTGTGAGC GCAGCAGTGGTGACATTGGAGATAGCTATGTGAG CTGGTACCAGCAACACTTGGGAAGACCCCCCATC AATGTGATCTATGCTGATGATCAAAGACCATCTGA AGTGTCTGATCGGTTCTCGGGCTCCATCGACACCT CCTCTAACTCAGCCTCACTGACCATCACTAATCTG CAGATGGATGATGCGGCCGACTACTTCTGTCAGT CTTACGATAGTAATATTGATTTTAACCCTGTTTTCG GTGGTGGAACCAAGCTCACTGTCCTA |
| 19F6-VH | 82 | DNA | GAGGTGCAGCTGGTGGAGTCTGGTGGAGGCTTAG TGCAGCCTGGAAGGTCTCTGAGACTCTCCTGTACA GCCTCAGGATTCACTTTCAGTAAATATGTCATGGC CTGGGTCCGCCAGGCTCCAACGAAGGGGCTGGA GTGGGTCGCATCCATTAATTATGATGGTAGTACCA CTTACTATCGAGACTCCGTGCAGGGCCGGTTCAC TCTCTCCAGAGATAATGCAAAAACCACCCTATACC TGCAAATGGACAGTCTGAGGTCTGAGGACACGGC CACTTATTACTGTGCAAGGCACCCTTTTAACAACTT CGGGATTTGGTTTGCTTACTGGGGCCAAGGCACT CTGGTCACTGTCTCTTCA |
| 19F6-CDR-L1 | 83 | DNA | GAGCGCAGCAGTGGTGACATTGGAGATAGCTATG TGAGC |
| 19F6-CDR-L2 | 84 | DNA | GCTGATGATCAAAGACCATCT |
| 19F6-CDR-L3 | 85 | DNA | CAGTCTTACGATAGTAATATTGATTTTAACCCTGTT |
| 19F6-CDR-H1 | 86 | DNA | AAATATGTCATGGCC |
| 19F6-CDR-H2 | 87 | DNA | TCCATTAATTATGATGGTAGTACCACTTACTATCGA GACTCCGTGCAGGGC |
| 19F6-CDR-H3 | 88 | DNA | CACCCTTTTAACAACTTCGGGATTTGGTTTGCTTAC |
| 19F6-VL | 89 | PRT | QFTLTQPKSVSGSLRSTITIPCERSSGDIGDSYVSWY QQHLGRPPINVIYADDQRPSEVSDRFSGSIDTSSNSA SLTITNLQMDDAADYFCQSYDSNIDFNPVFGGGTKLT VL |
| 19F6-VH | 90 | PRT | EVQLVESGGGLVQPGRSLRLSCTASGFTFSKYVMA WVRQAPTKGLEWVASINYDGSTTYYRDSVQGRFTL SRDNAKTTLYLQMDSLRSEDTATYYCARHPFNNFGI WFAYWGQGTLVTVSS |
| 19F6-CDR-L1 | 91 | PRT | ERSSGDIGDSYVS |
| 19F6-CDR-L2 | 92 | PRT | ADDQRPS |
| 19F6-CDR-L3 | 93 | PRT | QSYDSNIDFNPV |
| 19F6-CDR-H1 | 94 | PRT | KYVMA |
| 19F6-CDR-H2 | 95 | PRT | SINYDGSTTYYRDSVQG |
| 19F6-CDR-H3 | 96 | PRT | HPFNNFGIWFAY |
| 21F12-VL | 97 | DNA | GATGTCCGGATGACACAGTCTCCAGCTTCCCTGTC TGCATCTCTGGGAGAAACTGTCAACATCGAATGTC TAGCAAGTGAGGACATTTACAGTGATTTAGCATGG TATCAGCAGAAGCCAGGGAAATCTCCTCAGCTCCT GATCTATAATGCAAATAGTCTACAAAATGGGGTCC CTTCACGGTTTAGTGGCAGTGGTTCTGGCACGCA GTATTCTCTAAAAATATCCACCCTGCAATCTGAAGA TGTCGCGACTTATTTCTGTCAACAATATAGCAATTA TCGTCGGACGTTCGGTGGAGGCACCAAGCTGGAA ATCAAT |
| 21F12-VH | 98 | DNA | GAGGTGCAACTGGTGGAGTCTGGGGGAGGCCTA GTGCAGCCTGGAAGGTCTCTGAAACTATCCTGTGT AGCCTCTGGATTCACATTCAACAACCACTGGATGA CCTGGATCCGCCAGGCTCCAGGGAAGGGGCTGG AGTGGGTTGCATCCATTACTGATAATGGTGGTAGC ACTTACTATCCAGACTCTGTGAAGGGCCGATTCAC TATCTCCAGAGATAATGCAAAAAGCACCCTATACC TGCACATGAACAGTCTGAGGTCTGAGGACACGGC CACTTATTACTGTACAAGAGATCGGTATGACTCTG ATGGTTATTATTACGTGAGGTACTATGTTGTGGAC GCCTGGGGTCAAGGAGCTTCAGTCACTGTCTCCT CA |
| 21F12-CDR-L1 | 99 | DNA | CTAGCAAGTGAGGACATTTACAGTGATTTAGCA |
| 21F12-CDR-L2 | 100 | DNA | AATGCAAATAGTCTACAAAAT |
| 21F12-CDR-L3 | 101 | DNA | CAACAATATAGCAATTATCGTCGGACG |
| 21F12-CDR-H1 | 102 | DNA | AACCACTGGATGACC |
| 21F12-CDR-H2 | 103 | DNA | TCCATTACTGATAATGGTGGTAGCACTTACTATCC AGACTCTGTGAAGGGC |
| 21F12-CDR-H3 | 104 | DNA | GATCGGTATGACTCTGATGGTTATTATTACGTGAG GTACTATGTTGTGGACGCC |
| 21F12-VL | 105 | PRT | DVRMTQSPASLSASLGETVNIECLASEDIYSDLAWY QQKPGKSPQLLIYNANSLQNGVPSRFSGSGSGTQY SLKISTLQSEDVATYFCQQYSNYRRTFGGGTKLEIN |
| 21F12-VH | 106 | PRT | EVQLVESGGGLVQPGRSLKLSCVASGFTFNNHWMT WIRQAPGKGLEWVASITDNGGSTYYPDSVKGRFTIS RDNAKSTLYLHMNSLRSEDTATYYCTRDRYDSDGY YYVRYYVVDAWGQGASVTVSS |
| 21F12-CDR-L1 | 107 | PRT | LASEDIYSDLA |
| 21F12-CDR-L2 | 108 | PRT | NANSLQN |
| 21F12-CDR-L3 | 109 | PRT | QQYSNYRRT |
| 21F12-CDR-H1 | 110 | PRT | NHWMT |
| 21F12-CDR-H2 | 111 | PRT | SITDNGGSTYYPDSVKG |
| 21F12-CDR-H3 | 112 | PRT | DRYDSDGYYYVRYYVVDA |
| 34F8-VL (refers to an antibody according to the present invention) | 113 | DNA | GATGTTGTGTTGACACAGACTCCAGGTTCCCTGTC TGTCACACTTGGACAGCAAGTTTCTATATCCTGTA GGTCTAGTCAGAGCCTGGAAAGTCGTGATGGGAA CACTTATTTGGAATGGTACCTACAGAAGCCAGGCC AGTCTCCACAGGTCCTCCTCTATGGAGTTTCCAAC CGATTGTCTGGGGTCCCAGACAGGTTCCTTGGCA GAGGGTCAGGGGCAGATTTCACCCTCAAGATCAG CAGAGTAGAGCCTGAGGACTTGGGAGTTTATTACT GCTTCCAAGCTACACATGGTCCATTCACGTTCGGC TCAGGGACGAAGTTGGAAATGAAA |
| 34F8-VH (refers to an antibody according to the present invention) | 114 | DNA | CAGGTGCAGCTGAAGGAGTCAGGACCTGGCCTGG TGCAGCCCTCACAGACCCTGTCTCTCACCTGCACT GTCTCTGGGTTCTCATTAACCACCTATCATGTGCA CTGGGTTCGACAGCCTCCAGGAAAAGGTCTGGAG TGGATGGGAATAATGTGGAGAGATGGAGACACAT CATATAATTCAGTTCTCAAATCTCGACTGAGCATCA GCAGGGACATCTCCAAGAGCCAAGTTTTCTTAAAA ATGAGCAGTCTGCAAACTGAAGACACAGCCACTTA CTTCTGTGCCAGAGGGGGGACTCTTACAACTCCC TTTACTTACTGGGGCCAAGGCACTCTGGTCACTGT CTCTTCA |
| 34F8-CDR-L1 (refers to an antibody according to the present invention) | 115 | DNA | AGGTCTAGTCAGAGCCTGGAAAGTCGTGATGGGA ACACTTATTTGGAA |
| 34F8-CDR-L2 (refers to an antibody according to the present invention) | 116 | DNA | GGAGTTTCCAACCGATTGTCT |
| 34F8-CDR-L3 (refers to an antibody according to the present invention) | 117 | DNA | TTCCAAGCTACACATGGTCCATTCACG |
| 34F8-CDR-H1 (refers to an antibody according to the present invention) | 118 | DNA | ACCTATCATGTGCAC |
| 34F8-CDR-H2 (refers to an antibody according to the present invention) | 119 | DNA | ATAATGTGGAGAGATGGAGACACATCATATAATTC AGTTCTCAAATCT |
| 34F8-CDR-H3 (refers to an antibody according to the present invention) | 120 | DNA | GGGGGGACTCTTACAACTCCCTTTACTTAC |
| 34F8-VL (refers to an antibody according to the present invention) | 121 | PRT | DVVLTQTPGSLSVTLGQQVSISCRSSQSLESRDGNT YLEWYLQKPGQSPQVLLYGVSNRLSGVPDRFLGRG SGADFTLKISRVEPEDLGVYYCFQATHGPFTFGSGT KLEMK |
| 34F8-VH (refers to an antibody according to the present invention) | 122 | PRT | QVQLKESGPGLVQPSQTLSLTCTVSGFSLTTYHVHW VRQPPGKGLEWMGIMWRDGDTSYNSVLKSRLSISR DISKSQVFLKMSSLQTEDTATYFCARGGTLTTPFTY WGQGTLVTVSS |
| 34F8-CDR-L1 (refers to an antibody according to the present invention) | 123 | PRT | RSSQSLESRDGNTYLE |
| 34F8-CDR-L2 (refers to an antibody according to the present invention) | 124 | PRT | GVSNRLS |
| 34F8-CDR-L3 (refers to an antibody according to the present invention) | 125 | PRT | FQATHGPFT |
| 34F8-CDR-H1 (refers to an antibody according to the present invention) | 126 | PRT | TYHVH |
| 34F8-CDR-H2 (refers to an antibody according to the present invention) | 127 | PRT | IMWRDGDTSYNSVLKS |
| 34F8-CDR-H3 (refers to an antibody according to the present invention) | 128 | PRT | GGTLTTPFTY |
| 38E4-VL | 129 | DNA | GATATCCGGATGACACAGTCTCCAGCTTCCCTGTC TGCATCTCTGGGAGAAACTGTCAACATCGAATGTC TAGCAAGTGAGGACATTTACAGTGATTTAGCATGG TATCAGCAGAAGCCAGGGAAATCTCCACAACTCCT GATCTATAATGCAAATAGCGTGCAAAATGGGGTCC CTTCACGGTTTAGTGGCAGTGGATCTGGCACACA GTATTCTCTAAAAATAAACAGCCTGCAATCTGAAG ATGTCGCGACTTATTTCTGTCAACAGTTTAACAGTT ATCCGAACACGTTTGGAGCTGGGACCAAGCTGGA AATCAAA |
| 38E4-VH | 130 | DNA | GAGGTGCAACTTCAGGAGTCAGGACCTGGCCTTG TGAAACCCTCACAGTCACTCTCCCTCACCTGTTCT GTCTCTGGTTTCTCCATCACTAATAATTACTGGGG CTGGATCCGGAAGTTCCCAAGAAATAAAATGGAGT GGATTGGACACATAAGCTACAGTGGTAGCACTAAC TACAACCCATCTCTCAAAAGTCGCATCTCCATTACT AGAGACTCATCGAAGAGTCAGTTCTTCCTGCAGTT GAACTCTTTAACTACTGAGGACACAGCCACATATT ACTGTGCAAGAGGATCTTATTACTATAGCGCATCG GGCTACTTTGATTATTGGGGCCAAGGAATCACGGT CACAGTCTCCTCA |
| 38E4-CDR-L1 | 131 | DNA | CTAGCAAGTGAGGACATTTACAGTGATTTAGCA |
| 38E4-CDR-L2 | 132 | DNA | AATGCAAATAGCGTGCAAAAT |
| 38E4-CDR-L3 | 133 | DNA | CAACAGTTTAACAGTTATCCGAACACG |
| 38E4-CDR-H1 | 134 | DNA | AATAATTACTGGGGC |
| 38E4-CDR-H2 | 135 | DNA | CACATAAGCTACAGTGGTAGCACTAACTACAACCC ATCTCTCAAAAGT |
| 38E4-CDR-H3 | 136 | DNA | GGATCTTATTACTATAGCGCATCGGGCTACTTTGA TTAT |
| 38E4-VL | 137 | PRT | DIRMTQSPASLSASLGETVNIECLASEDIYSDLAWYQ QKPGKSPQLLIYNANSVQNGVPSRFSGSGSGTQYSL KINSLQSEDVATYFCQQFNSYPNTFGAGTKLEIK |
| 38E4-VH | 138 | PRT | EVQLQESGPGLVKPSQSLSLTCSVSGFSITNNYWG WIRKFPRNKMEWIGHISYSGSTNYNPSLKSRISITRD SSKSQFFLQLNSLTTEDTATYYCARGSYYYSASGYF DYWGQGITVTVSS |
| 38E4-CDR-L1 | 139 | PRT | LASEDIYSDLA |
| 38E4-CDR-L2 | 140 | PRT | NANSVQN |
| 38E4-CDR-L3 | 141 | PRT | QQFNSYPNT |
| 38E4-CDR-H1 | 142 | PRT | NNYWG |
| 38E4-CDR-H2 | 143 | PRT | HISYSGSTNYNPSLKS |
| 38E4-CDR-H3 | 144 | PRT | GSYYYSASGYFDY |
| 42A5-VL | 145 | DNA | GACGTGGTCTTGACCCAAACCCCTGGATCACTTAG CGTGACACTGGGCGATCCAGCATCAATGTCCTGC AGAAGCTCCCAGTCCTTGGAGAGTAGCGACGGCA ACACATACCTCGAGTGGTATCTGCAGAAATCCGG GCAGTCCCCACAGCTGCTGATCTACGGCGTGAGT AACAGGTTCAGCGGGGTGCCTGATAGGTTCGCCG GCAGCGGGTCCGGGACAGATTTTACTCTCAAGATT AGCCGCGTCGAACCCGAGGACCTGGGCGTGTACT ACTGTTTTCAGGCCACTCGGGACCCCTTTACTTTC GGGAGCGGGACAAAGCTGGAGATTAAT |
| 42A5-VH | 146 | DNA | CAGGTCCAGCTTAAAGAGTCCGGACCTGGACTTG TGCAGCCATCCCAGACCTTGTCCTTGACCTGCACC GTGTCAGGGTTCTCTCTCACCAGTTACCACCTGCA TTGGATCAGGCAGCCTCCCGGCAAGGGGCTGGAA TGGATGGGGCTGATGTGGAGAGATGGGGATACAT CTTACAACAGCAGGCTGAAGAGCCGGCTGAGCAT TACACGGGACACCAGCAAGTCCCAGGTGTTCCTC AAGATGAGCGGGCTCCAAACTGAGGACACAGCTA CATACTACTGTGCACGCGGCATGACACTCGCCAC TCCCTTTCTGTATTGGGGCCAGGGCACTCTGGTCA CTGTGTCCTCA |
| 42A5-CDR-L1 | 147 | DNA | AGAAGCTCCCAGTCCTTGGAGAGTAGCGACGGCA ACACATACCTCGAG |
| 42A5-CDR-L2 | 148 | DNA | GGCGTGAGTAACAGGTTCAGC |
| 42A5-CDR-L3 | 149 | DNA | TTTCAGGCCACTCGGGACCCCTTTACT |
| 42A5-CDR-H1 | 150 | DNA | AGTTACCACCTGCAT |
| 42A5-CDR-H2 | 151 | DNA | CTGATGTGGAGAGATGGGGATACATCTTACAACAG CAGGCTGAAGAGC |
| 42A5-CDR-H3 | 152 | DNA | GGCATGACACTCGCCACTCCCTTTCTGTAT |
| 42A5-VL | 153 | PRT | DVVLTQTPGSLSVTLGDPASMSCRSSQSLESSDGNT YLEWYLQKSGQSPQLLIYGVSNRFSGVPDRFAGSG SGTDFTLKISRVEPEDLGVYYCFQATRDPFTFGSGT KLEIN |
| 42A5-VH | 154 | PRT | QVQLKESGPGLVQPSQTLSLTCTVSGFSLTSYHLHW IRQPPGKGLEWMGLMWRDGDTSYNSRLKSRLSITR DTSKSQVFLKMSGLQTEDTATYYCARGMTLATPFLY WGQGTLVTVSS |
| 42A5-CDR-L1 | 155 | PRT | RSSQSLESSDGNTYLE |
| 42A5-CDR-L2 | 156 | PRT | GVSNRFS |
| 42A5-CDR-L3 | 157 | PRT | FQATRDPFT |
| 42A5-CDR-H1 | 158 | PRT | SYHLH |
| 42A5-CDR-H2 | 159 | PRT | LMWRDGDTSYNSRLKS |
| 42A5-CDR-H3 | 160 | PRT | GMTLATPFLY |
| 42F4-VL | 161 | DNA | GATATCCGGATGACACAGTCGCCAGCTTCCCTGT CTGCATCTCTGGGAGAAACTGTCAACATCGAATGT CTAGCAAGTGAGGACATTCACAGTGATTTAGCATG GTATCAGCAGAAGCCAGGGAAATCTCCTCAGCTC CTGATCTATAATGCAAATAGCTTGCAAAATGGGGT CCCTTCACGGTTCAGTGGCAGTGGATCTGGCACA CAGTATTCTCTAAAAATAACCAGCCTGCAATCTGA AGATGTCGCGACTTATTTCTGTCAACAATATACCAA CTATCCGAACACGTTTGGAGCGGGGACCAAGCTG GAAATCAAT |
| 42F4-VH | 162 | DNA | GAGGTGCAGCTTCAGGAGTCAGGACCTGGCCTTG TGAAACCCTCACAGTCACTCTCCCTCACCTGTTCT GTCACTGGTTACTCCATCACTAATCATTACTGGGG CTGGATCCGGAAATTCCCAGGAAATAAAATGGAGT GGATTGGACACATAAGCAACAGTGGTGGCACTAA CTACAACCCATCACTCAAAAGTCGAATCTCCATTA CTAGAGACACATCGAAGAATCAGTTCTTCCTGCAG TTGAAGTCTGTAACTACTGAGGACACAGCCACATA TTACTGTACAAGAGGATCTTATTACTATAGCGCATC GGGCTACTTTGATTACTGGGGCCAAGGAGTCCTG GTCACAGTCTCCTCC |
| 42F4-CDR-L1 | 163 | DNA | CTAGCAAGTGAGGACATTCACAGTGATTTAGCA |
| 42F4-CDR-L2 | 164 | DNA | AATGCAAATAGCTTGCAAAAT |
| 42F4-CDR-L3 | 165 | DNA | CAACAATATACCAACTATCCGAACACG |
| 42F4-CDR-H1 | 166 | DNA | AATCATTACTGGGGC |
| 42F4-CDR-H2 | 167 | DNA | CACATAAGCAACAGTGGTGGCACTAACTACAACCC ATCACTCAAAAGT |
| 42F4-CDR-H3 | 168 | DNA | GGATCTTATTACTATAGCGCATCGGGCTACTTTGA TTAC |
| 42F4-VL | 169 | PRT | DIRMTQSPASLSASLGETVNIECLASEDIHSDLAWYQ QKPGKSPQLLIYNANSLQNGVPSRFSGSGSGTQYSL KITSLQSEDVATYFCQQYTNYPNTFGAGTKLEIN |
| 42F4-VH | 170 | PRT | EVQLQESGPGLVKPSQSLSLTCSVTGYSITNHYWG WIRKFPGNKMEWIGHISNSGGTNYNPSLKSRISITRD TSKNQFFLQLKSVTTEDTATYYCTRGSYYYSASGYF DYWGQGVLVTVSS |
| 42F4-CDR-L1 | 171 | PRT | LASEDIHSDLA |
| 42F4-CDR-L2 | 172 | PRT | NANSLQN |
| 42F4-CDR-L3 | 173 | PRT | QQYTNYPNT |
| 42F4-CDR-H1 | 174 | PRT | NHYWG |
| 42F4-CDR-H2 | 175 | PRT | HISNSGGTNYNPSLKS |
| 42F4-CDR-H3 | 176 | PRT | GSYYYSASGYFDY |
| 45H1-VL | 177 | DNA | GATATCCGGATGACACAGTCTCCAGCTTCCCTGTC TGCATCTCTGGGAGAAACTGTCAACATCGGATGTC TAGCAAGTGAGGACATTTACAGTGATTTAGCATGG TATCAGCAGAAGCCAGGGAAGTCTCCTCAGCTCC TGATCTATAATGCAAATAACTTGCAAAATGGGGTC CCTTCACGGTTTAGTGGCAGTGGATCTGGCACAC AATATTCTCTAAAAATAAACAGCCTGCAATCTGAAG ATGTCGCGACTTATTTCTGTCAACAATATAACAGTT ATCCGAACACGTTTGGAGCTGGGACCAAGCTGGA AATAAAA |
| 45H1-VH | 178 | DNA | GAGGTGCAGCTTCAGGAGTCAGGACCTGGCCTTG TGAAACCCTCACAGTCACTCTCCCTCATTTGTTCT |
| | | | GTCACTGGTTACTCCATCACTACAACTTACTGGGG CTGGATCCGGAAGTTCCCAGGAAATAAAATGGAGT GGATTGGACACATAAGTAACAGTGGTAGTACTAAT TACAACCCATCTCTCAAAAGTCGAATCTCCGTTAC TAGAGACACATCGACGAATCAGTTCTTCCTGCAGT TGAACTCTGTAACTACTGAGGACACAGCCACATAT TACTGTGCAAGAGGATCTTATTACTATAGCGCGTC GGGCTACTTTGATTACTGGGGCCACGGAGTCATG GTCACAGTCTCCTCA |
| 45H1-CDR-L1 | 179 | DNA | CTAGCAAGTGAGGACATTTACAGTGATTTAGCA |
| 45H1-CDR-L2 | 180 | DNA | AATGCAAATAACTTGCAAAAT |
| 45H1-CDR-L3 | 181 | DNA | CAACAATATAACAGTTATCCGAACACG |
| 45H1-CDR-H1 | 182 | DNA | ACAACTTACTGGGGC |
| 45H1-CDR-H2 | 183 | DNA | CACATAAGTAACAGTGGTAGTACTAATTACAACCC ATCTCTCAAAAGT |
| 45H1-CDR-H3 | 184 | DNA | GGATCTTATTACTATAGCGCGTCGGGCTACTTTGA TTAC |
| 45H1-VL | 185 | PRT | DIRMTQSPASLSASLGETVNIGCLASEDIYSDLAWYQ QKPGKSPQLLIYNANNLQNGVPSRFSGSGSGTQYSL KINSLQSEDVATYFCQQYNSYPNTFGAGTKLEIK |
| 45H1-VH | 186 | PRT | EVQLQESGPGLVKPSQSLSLICSVTGYSITTTYWGWI RKFPGNKMEWIGHISNSGSTNYNPSLKSRISVTRDT STNQFFLQLNSVTTEDTATYYCARGSYYYSASGYFD YWGHGVMVTVSS |
| 45H1-CDR-L1 | 187 | PRT | LASEDIYSDLA |
| 45H1-CDR-L2 | 188 | PRT | NANNLQN |
| 45H1-CDR-L3 | 189 | PRT | QQYNSYPNT |
| 45H1-CDR-H1 | 190 | PRT | TTYWG |
| 45H1-CDR-H2 | 191 | PRT | HISNSGSTNYNPSLKS |
| 45H1-CDR-H3 | 192 | PRT | GSYYYSASGYFDY |
| 14E11-VL (control) | 193 | PRT | DIVMTQSHKFMSTSVGDRVSITCKASQDVSTAVAWY QQKPGQSPKLLIYLTSYRNTGVPDRFTGSGSGTDFT FTISSVQAEDLAVYYCQQHYKTPYSFGGGTKLERLR |
| 14E11-VH (control) | 194 | PRT | QVQLEESGPGLVAPSQSLSITCTVSGFSLTGYGIYW VRQPPGKGLEWLGMIWGDGRTDYNSALKSRLSISK DNSKSQVFLKMNSLQTDDTARYYCARDYYGSKDYW GQGTTLTVS |
| 1A6-VL (control) | 195 | PRT | DIVLTQSPASLAVSLGQRATISCKASQSVDYDGDSYL NWYQQKPGQPPKLLIYAASNLESGIPARFSGSGSGT DFTLNIHPVEEEDAATYYCQQSNGDPWTFGGGTKLE IK |
| 1A6-VH (control) | 196 | PRT | QVTLKESGPGILKPSQTLSLTCSFSGFSLSTSGMGV GWIRQPSGKGLEWLAHIWWDDDKYYNPSLKSQLTIS KDTSRNQVFLKITSVDAADTATYYCARKRSSVVAHY YAMDYWGQGTSVTVS |

Provided in certain embodiments herein are humanized anti-FXI and/or anti-FXla antibodies. Various techniques are known in the art for humanizing antibodies from non-human species such that the antibodies are modified to increase their similarity to antibodies naturally occurring in humans. Six CDRs are present in each antigen binding domain of a natural antibody. These CDRs are short, non-contiguous sequences of amino acids that are specifically positioned to form the antigen binding domain as the antibody assumes its three-dimensional configuration. The remainder of the amino acids in the antigen binding domains, referred to as "framework" regions, show less inter-molecular variability and form a scaffold to allow correct positioning of the CDRs.

For example, humanization of the antibodies disclosed herein can be accomplished by CDR grafting of monoclonal antibodies produced by immunizing mice or rats. The CDRs of a mouse monoclonal antibody can be grafted into a human framework, which is subsequently joined to a human constant region to obtain a humanized antibody. Briefly, the human germline antibody sequence database, the protein data bank (PDB), the INN (International Nonproprietary Names) database, and other suitable databases can be searched and the most similar frameworks to the antibodies can be identified by the search. In addition, some back mutations to the donor residues are made in the human acceptor frameworks. In some embodiments, the variable regions are linked to a human IgG constant region. For example, human IgG1, IgG2, IgG3 and IgG4 Fc domains can be used. It is within the purview of one of ordinary skill in the art to humanize a monoclonal antibody produced by a non-human species based on the existing technology.

The sequences of the variable regions of several example humanized antibodies are shown in Table 2 below.

**Table 2: Sequences of humanized antibodies**

| **Description** | **SEQ ID NO** | **Type** | **Sequence (with mutations highlighted)** |
|---|---|---|---|
| h-19F6-VL | 197 | PRT | QFQLTQPSSVSASVGDRVTITCERSSGDIGDSYVSWY QQKPGQPPKNVIYADDQRPSGVPDRFSGSIDGSGNS ASLTISSLQAEDAADYFCQSYDSNIDFNPVFGGGTKLE VK |
| h-19F6-VH | 198 | PRT | EVQLVESGGGLVQPGRSLRLSCTASGFTFSKYAMSW VRQAPGKGLEWVSAINYDGSTTYYADSVKGRFTLSRD NSKNTLYLQMNSLRAEDTAVYYCARHPFNNFGIWFAY WGQGTLVTVS |
| h-34F8-VL | 199 | PRT | DVVLTQTPLSLPVTPGEPASISCRSSQSLESRDGNTYL EWYLQKPGQSPQVLLYGVSNRLSGVPDRFSGSGSGT DFTLKISRVEAEDVGVYYCFQATHGPFTFGQGTKLEIK RT |
| h-34F8-VH | 200 | PRT | QVQLQESGPGLVKPSETLSLTCTVSGFSLTTYHVHWV RQPPGKGLEWIGIMWRDGDTYYNSSLKSRVTISRDTS KNQVSLKLSSVTAADTAVYFCARGGTLTTPFTYWGQG TLVTVSS |
| h-42A5-VL | 201 | PRT | DVVLTQTPLSLSVTPGQPASISCRSSQSLESSDGNTYL EWYLQKPGQSPQLLIYGVSNRFSGVPDRFSGSGSGTD FTLKISRVEAEDLGVYYCFQATRDPFTFGQGTKLEIKR T |
| h-42A5-VH | 202 | PRT | QVTLKESGPVLVKPTETLTLTCTVSGFSLTSYHLHWIR QPPGKALEWMGLMWRDGDTSYNSRLKSRLTISRDTS KSQVVLTMTNMDPVDTATYYCARGMTLATPFLYWGQ GTLVTVSS |

The antibodies provided herein include variants of the sequences disclosed herein that contain one or more mutations in their amino acid sequences while retaining binding affinity for FXI, FXla, and/or a fragment thereof (e.g., a fragment comprising the A3 domain). In some embodiments, the antibodies include a variable region having an amino acid sequence that is at least 85%, at least 90%, at least 95%, at least 98%, or at least 99% identical to a sequence selected from the group consisting of SEQ ID NOs: 9, 10, 25, 26, 41, 42, 57, 58, 73, 74, 89, 90, 105, 106, 121, 122, 137, 138, 153, 154, 169, 170, 185, 186, and 197-202, or a fragment thereof that retains binding affinity for FXI, FXla, and/or a fragment thereof.

Also included in this disclosure are variants of nucleic acids encoding antibodies that bind to FXI, FXla, and/or a fragment thereof (e.g., a fragment comprising the A3 domain). In some embodiments, the nucleic acids encoding the antibodies include a variable region having a nucleic acid sequence that is at least 85%, at least 90%, at least 95%, at least 98%, or at least 99% identical to a sequence selected from the group consisting of SEQ ID NOs: 1, 2, 17, 18, 33, 34, 49, 50, 65, 66, 81, 82, 97, 98, 113, 114, 129, 130, 145, 146, 161, 162, 177, and 178, or a fragment thereof that encodes a polypeptide with binding affinity for FXI, FXla, and/or a fragment thereof.

### Pharmaceutical Compositions

The antibodies disclosed herein can be formulated into pharmaceutical compositions. The pharmaceutical compositions may further comprise one or more pharmaceutically acceptable carriers, excipients, preservatives, or a combination thereof. The pharmaceutical compositions can have various formulations, e.g., injectable formulations, lyophilized formulations, liquid formulations, etc. Depending on the formulation and administration route, one would select suitable additives, such as adjuvants, carriers, excipients, preservatives. See, for example, Wang et al., J. Pharm. Sciences 96(1): 1-26 (2007).

The pharmaceutical composition can be included in a kit with an instruction for using the composition.

### Methods of Treatment

Provided herein is a method of treating and/or preventing thrombosis in a subject suffering from thrombosis and/or at an elevated risk of developing thrombosis. Also provided is a method of inhibiting the formation of blood clots in a subject. These methods entail administering a therapeutically effective amount of an anti-FXI and/or FXla antibody provided herein to intervene in the intrinsic pathway. In some embodiments, these methods comprise administering a pharmaceutical composition comprising an anti-FXI and/or anti-FXla antibody as provided herein to the subject.

The methods disclosed herein can be used to prevent and/or treat complications or conditions associated with thrombosis in a subject in need thereof. Thrombosis causes or is associated with a number of complications or conditions, such as embolic stroke, venous thrombosis such as venous thromboembolism (VTE), deep vein thrombosis (DVT), and pulmonary embolism (PE), arterial thrombosis such as acute coronary syndrome (ACS), coronary artery disease (CAD), and peripheral artery disease (PAD). Other conditions associated with thrombosis include, for example, high risk of VTE in surgical patients, immobilized patients, patients with cancer, patients with heart failure, pregnant patients, or patients having other medical conditions that may cause thrombosis. The methods disclosed herein relate to a preventive anticoagulant therapy, that is, thromboprophylaxis. These methods entail administering to a subject suffering from a thrombosis-related complication disclosed above a therapeutically effective amount of an anti-FXI and/or FXla antibody as disclosed herein or a therapeutically effective amount of a pharmaceutical composition comprising the anti-FXI and/or FXla antibody. The antibody or pharmaceutical composition can be administered either alone or in combination with any other therapy for treating or preventing the thrombosis-related complications or conditions.

Also provided is a method of treating and/or preventing sepsis in a subject in need thereof. It was attempted to administer anticoagulants to sepsis patients to improve mortality or morbidity. However, the attempt was unsuccessful due to the undesired bleeding caused by anticoagulants. The antibodies disclosed herein can be used as a secondary therapy in combination with other therapeutic agents for treating sepsis, such as antibiotics.

As used herein, the term "subject" refers to mammalian subject, preferably a human. A "subject in need thereof" refers to a subject who has been diagnosed with thrombosis or complications or conditions associated with thrombosis, or is at an elevated risk of developing thrombosis or complications or conditions associated with thrombosis. The phrases "subject" and "patient" are used interchangeably herein.

The terms "treat," "treating," and "treatment" as used herein with regard to a condition refers to alleviating the condition partially or entirely, preventing the condition, decreasing the likelihood of occurrence or recurrence of the condition, slowing the progression or development of the condition, or eliminating, reducing, or slowing the development of one or more symptoms associated with the condition. With regard to thrombosis and/or complications or conditions associated with thrombosis, "treating" may refer to preventing or slowing the existing blood clot from growing larger, and/or preventing or slowing the formation of blood clot. In some embodiments, the term "treat," "treating," or "treatment" means that the subject has a reduced number or size of blood clots comparing to a subject without being administered with the antibodies or functional fragments thereof. In some embodiments, the term "treat," "treating," or "treatment" means that one or more symptoms of thrombosis and/or thrombosis-related conditions or complications are alleviated in a subject receiving an antibody or pharmaceutical composition as disclosed herein comparing to a subject who does not receive such treatment.

A "therapeutically effective amount" of an antibody or pharmaceutical composition as used herein is an amount of the antibody or pharmaceutical composition that produces a desired therapeutic effect in a subject, such as treating and/or preventing thrombosis. In certain embodiments, the therapeutically effective amount is an amount of the antibody or pharmaceutical composition that yields maximum therapeutic effect. In other embodiments, the therapeutically effective amount yields a therapeutic effect that is less than the maximum therapeutic effect. For example, a therapeutically effective amount may be an amount that produces a therapeutic effect while avoiding one or more side effects associated with a dosage that yields maximum therapeutic effect. A therapeutically effective amount for a particular composition will vary based on a variety of factors, including but not limited to the characteristics of the therapeutic composition (e.g., activity, pharmacokinetics, pharmacodynamics, and bioavailability), the physiological condition of the subject (e.g., age, body weight, sex, disease type and stage, medical history, general physical condition, responsiveness to a given dosage, and other present medications), the nature of any pharmaceutically acceptable carriers, excipients, and preservatives in the composition, and the route of administration. One skilled in the clinical and pharmacological arts will be able to determine a therapeutically effective amount through routine experimentation, namely by monitoring a subject's response to administration of the antibody or the pharmaceutical composition and adjusting the dosage accordingly. For additional guidance, see, e.g., Remington: The Science and Practice of Pharmacy, 22nd Edition, Pharmaceutical Press, London, 2012, and Goodman & Gilman's The Pharmacological Basis of Therapeutics, 12th Edition, McGraw-Hill, New York, NY, 2011.

In some embodiments, a therapeutically effective amount of an antibody disclosed herein is in the range from about 0.01 mg/kg to about 30 mg/kg, from about 0.1 mg/kg to about 10 mg/kg, from about 1 mg/kg to about 5 mg/kg.

It is within the purview of one of ordinary skill in the art to select a suitable administration route, such as subcutaneous administration, intravenous administration, intramuscular administration, intradermal administration, intrathecal administration, or intraperitoneal administration. For treating a subject in need thereof, the antibody or pharmaceutical composition can be administered continuously or intermittently, for an immediate release, controlled release or sustained release. Additionally, the antibody or pharmaceutical composition can be administered three times a day, twice a day, or once a day for a period of 3 days, 5 days, 7 days, 10 days, 2 weeks, 3 weeks, or 4 weeks. The antibody or pharmaceutical composition may be administered over a predetermined time period. Alternatively, the antibody or pharmaceutical composition may be administered until a particular therapeutic benchmark is reached. In certain embodiments, the methods provided herein include a step of evaluating one or more therapeutic benchmarks to determine whether to continue administration of the antibody or pharmaceutical composition.

### Method of Producing Antibodies

Also provided herein are methods of producing the anti-FXI and/or anti-FXla antibodies disclosed herein. In certain embodiments, these methods entail the steps of cloning a nucleic acid encoding an anti-FXI and/or anti-FXla antibody into a vector, transforming a host cell with the vector, and culturing the host cell to express the antibody. The expressed antibody can be purified from the host cell using any known technique. Various expression vectors such as pTT5 vector, and pcDNA3 vector, as well as various host cell lines such as CHO cells (e.g. CHO-K1 and ExpiCHO), and HEK293T cells, can be used.

Also encompassed by this disclosure are antibodies produced by the method disclosed above. The antibodies may have been subjected to one or more post-translational modifications.

The following examples are provided to better illustrate the embodiments and are not to be interpreted as limiting the scope of any claimed embodiment.

### Examples

### Example 1: Generation and sequencing of anti-FXI antibodies

BALB/c mice and Wistar rats were immunized with human FXI, and splenocytes from the animals with good immune response were collected for the preparation of hybridomas, which were subjected to subcloning by limiting dilution. Twelve monoclonal hybridoma clones expressing desired anti-FXI antibodies 3G12, 5B2, 7C9, 7F1, 13F4, 19F6, 21F12, 34F8, 38E4, 42A5, 42F4, and 45H1 were obtained by using capture ELISA and functional screening, wherein the notifier 34F8 refers to an antibody according to the present invention and the notifiers 3G12, 5B2, 7C9, 7F1, 13F4, 19F6, 21F12, 38E4, 42A5, 42F4, and 45H1 refer to antibodies not in accordance with the present invention.

To determine the amino acid and nucleotide sequences of the variable region of the light (V_{L}) and heavy chain (V_{H}) of these antibodies, cDNAs encoding V_{L} and V_{H} were cloned from the corresponding hybridoma cells by standard RT-PCR procedures. The V_{L} and V_{H} sequences of exemplary antibodies, including the sequences of CDRs, are shown in Table 1.

### Example 2: Determination of anti-coagulation activity in human plasma using activated partial thromboplastin time (APTT) assay and prothrombin time (PT) assay

APTT assay measures the activity of the intrinsic and common pathways of coagulation; whereas PT assay measures the activity of the extrinsic and common pathways of coagulation. The antibodies tested in this experiment were 19F6, 34F8, 42A5, 1A6 and 14E11. The sequences of the variable regions of the control antibodies were obtained from U.S. Patent No. 8,388,959 and US2013171144
and reformatted to IgG4. These antibodies were then expressed using ExpiCHO cell system. Standard human plasma purchased from Symens Inc. was mixed with equal volume of various antibodies at various concentrations from 0 to 400 nM for 5 minutes before being tested on a CA600 analyzer. In the APTT assay, 50 µL of the plasma-antibody mixture and 25 µL of APTT reagent (SMN 10445709, Symens Inc.) were mixed at 37 °C for 4 min. Then 25 µL of CaCl₂ Solution (25 mM, SMN 10446232, Symens Inc.) was added and time to clot formation was determined. In the PT assay, 50 µL of the plasma-antibody mixture was mixed with an equal volume of PT reagent (SMN 10446442, Symens Inc.) at 37 °C and time to clot formation was determined.

As shown in Figure 1, all antibodies tested increased APTT in a concentration-dependent manner at a relatively low concentration, for example, up to 100 nM (or up to 200 nM for 14E11); whereas none of these antibodies had a significant effect on PT (data not shown). These results indicate that all of the antibodies tested inhibited the intrinsic pathway of coagulation but not the extrinsic pathway.

### Example 3: Determination of the anti-coagulation activity in the plasma of non-human species using activated partial thromboplastin time (APTT) assay

Effects of various antibodies, including 19F6, 34F8 and 42A5, on coagulation were assessed in the mouse, rat, and monkey plasma using the same method as described in Example 2. None of the antibodies tested had any effect on APTT in the mouse and rat plasma, but all of them, at a relatively low concentration, concentrationdependently, increased APTT in the monkey plasma as shown in Figure 2, indicating that the antibodies tested had cross-activity with monkey FXI/FXIa, but not with mouse or rat FXI/FXIa.

### Example 4: Humanization of anti-FXI antibodies

The use of murine monoclonal antibodies directly as therapeutics has been hindered by the short half-life and the elicitation of the human anti-murine antibody responses. One solution to this problem is to humanize the murine antibodies. Some antibodies were subjected to humanization by CDR grafting. Suitable human acceptor frameworks for both V_{L} and V_{H} of each murine antibody were identified and varying numbers of back mutations were introduced to the selected human frameworks to maintain the structure and/or function of the resulting antibody. If the affinity and function of these humanized antibodies were not substantially inferior to the corresponding unmodified antibodies, the modified antibodies were considered successfully humanized. Three humanized V_{H} and V_{L} sequences of 19F6, 34F8 and 42A5, described as h-19F6, h-34F8, and h-42A5, respectively, are shown in Table 2.

### Example 5: Determination of the affinity of anti-FXI antibodies to human FXI

The affinity of anti-FXI/FXla antibodies to FXI/FXla were determined using surface plasmon resonance (SPR) technology performed on the BIAcore T200 instrument. The humanized antibodies were constructed by linking the variable regions of the antibodies disclosed herein to human IgG4 Fc domain and the recombinants were expressed in CHO cells. These antibodies were captured onto a Biacore CM5 sensor chip that was pre-immobilized with an anti-human IgG antibody.

Then different concentrations of the purified antigen FXI or FXla (0.005-1 µg/mL) were allowed to flow through the CM5 chip for 180 s for association with the anti-FXI/FXla antibody, followed by a time of 1800 s for dissociation. The binding data was collected and the affinity between FXI/FXla and the test antibodies was analyzed using the Biacore Evaluation Software provided by GE Healthcare. The SPR sensorgrams of FXI/FXla binding to immobilized h-19F6, h-34F8, and h-42A5 are shown in Figure 3. As shown in Figure 3, the response (RU) for each antibody became higher with escalating concentrations of FXI or FXIa. The dissociation constants (K_{D}) of h-19F6, h-34F8, and h-42A5 to FXI and FXla were calculated and detailed in Table 3. The affinities of each antibody to FXI and FXla are considered to be the same since the difference between them is less than 10 times.

**Table 3: K_{D} values of the antibodies to FXI and FXIa**

| **Antibody** | **K_{D} (pM)** | |
|---|---|---|
| | **FXI** | **FXIa** |
| h-19F6 | 22.2 | 25.8 |
| h-34F8 | 19.5 | 3.85 |
| h-42A5 | 35.7 | 8.14 |

### Example 6: Determination of the binding site of anti-FXI antibodies on FXI

The binding sites of 19F6 and 42A5 on FXI were determined using the SPR technology. Briefly, human IgG capture antibody was pre-immobilized on a Biacore CM5 sensor chip, and recombinant h-19F6 or h-42A5 was captured by flowing through the chip. An equal amount (15 relative units) of h-19F6 and h-42A5 was captured through adjustment of the antibody flowing time. Then wild type FXI or chimeric FXI in which individual apple domain was replaced with the corresponding domain from the human prekallikrein (FXI/PK chimeras) was allowed to flow through the chip for 180 seconds for association with h-19F6 or h-42A5, followed by a time period of 1800 seconds for dissociation. The binding data was analyzed in a high-performance kinetic mode as only one concentration of FXI, wild-type or chimeric, was tested in the SPR assay. Results showed that both h-19F6 and h-42A5 bound FXI as well as FXI/PK chimeras except when the A3 domain of FXI was replaced with the corresponding PK domain, indicating that part or the complete epitope of h-19F6 and h-42A5 on FXI is located in the A3 domain.

### Example 7: Functional neutralization of FXIa by the antibodies

Human FXla activity was determined by measuring the cleavage of a specific, chromogenic substrate, S-2366 (Diapharma Inc.). For testing the inhibitory activity of the antibodies, antibodies h-19F6, h-34F8 and h-42A5 were pre-incubated for 5 minutes at room temperature with a final concentration of 5 nM of FXla in PBS (phosphate buffer saline). Then an equal volume of 1 mM of S-2366 was added to initiate the FXla cleavage reaction and changes in absorbance at 405 nm was monitored continuously using a M5^{e} plate reader (Molecular Devices Inc.). Data were analyzed using the GraphPad Prism software and are shown in Figure 4. The calculated apparent Ki for h-19F6, h-34F8, and h-42A5 are 0.67, 2.08, and 1.43 nM, respectively. Therefore, all three antibodies tested exhibited satisfying inhibitory effects on FXla at a relatively low concentration.

### Example 8: Inhibition of FXIa-mediated FIX activation by the antibodies

Human FIX (200 nM) was incubated with FXla (5 nM) in PBS with 5 mM CaCl₂ at room temperature with 1 µM control IgG or h-19F6 or h-42A5. At 0, 15, 30, 45, and 60 min intervals, 50-µL samples were collected into dodecyl sulfate sample buffer. Samples were size-fractionated on 10% non-reducing gels and transferred to polyvinylidene fluoride membranes. Western blotting was performed to determine the FIX as well as FIXa levels using goat anti- human FIX IgG (Affinity Biologicals). As shown in Figure 5, both h-19F6 and h-42A5 inhibit FIXa formation induced by FXI as compared to the control.

### Example 9: Evaluation of the effects of anti-FXI antibodies on clotting time in cynomolgus monkeys

Cynomolgus monkeys were intravenously administered with indicated doses of various antibodies. Blood from the superficial veins of the upper limb was collected at pre-dose and at 0.5, 1, 3, 6, 12, and 24 hours post-dose, and citrated plasma was prepared for APTT and PT determination. In the APTT test, 50 µL of diluted plasma sample and 25 µL of APTT reagent (SMN 10445709, Symens Inc.) were mixed and incubated at 37 °C for 4 min. Then 25 µL of CaCl₂ Solution (25 mM, SMN 10446232, Symens Inc.) was added and time to clot formation was determined. In the PT test, 50 µL of diluted plasma sample was mixed with equal volume of PT reagent (SMN 10446442, Symens Inc.) and incubated at 37°C and time to clot formation was determined. All three antibodies tested demonstrated dose-dependently increased APTT as shown in Figure 6 and none of them affected PT as shown in Figure 7.

### Example 10: Evaluation of the effects of anti-FXI antibodies in arteriovenous (AV) shunt thrombosis and tail vein bleeding models in cynomolgus monkeys

Both thrombosis and bleeding time were assessed in the same animal for multiple doses of each antibody tested. The antibodies included in this experiment were h-34F8, h-19F6, and h42A5. Briefly, bleeding time and thrombosis were sequentially evaluated at pre-dose and 30 minutes following each administration of the antibody. The bleeding/thrombosis assessments were conducted four times: pre-dose and post-dose at three escalating dose levels (0.1, 0.3 and 1 mg/kg).

For AV shunt thrombosis, a shunt device containing a pre-weighed 10-cm long silk thread was applied to connecting the femoral arterial and femoral venous cannulae, and blood was allowed to flow through the shunt for 10 minutes. Then the thread was removed from the shunt and weighed again. Clot weight on the thread was calculated as the difference of the thread weight before and after blood flow.

For bleeding time evaluation, a 2-mL syringe was inserted into the tail vein of the animals. When the volume of blood in the syringe stopped increasing, the elapsed time was recorded manually as the bleeding time.

All antibodies dose-dependently reduced the thrombus weight as shown in Figure 8 and none of them prolonged the tail vein bleeding time as shown in Figure 9.

### Example 11: Evaluation of the effects of anti-FXI antibodies on ferric chlorideinduced artery thrombosis and template bleeding time in cynomolgus monkeys

Cynomolgus monkeys were pre-anesthetized with 1.5 mg/kg of Zoletil, intubated, and ventilated with a respirator. Anesthesia was maintained with isoflurane. The blood pressure, heart rate, and body temperature were monitored throughout the entire procedure. The antibodies tested, including h-34F8, h-19F6, and h-42A5, or the vehicle control were administered through limb vein by injection 2 hours before FeCl₃ application. The left femoral artery was exposed and isolated via blunt dissection. A Doppler flow probe was set up on the artery and the blood flow was continuously recorded. Before applying FeCl₃, the blood flow was measured for at least 5 minutes. Then two pieces of filter paper pre-soaked with FeCl₃ were applied to the adventitial surface of the vessel upstream from the probe for 10 minutes. After the filter paper was removed, the site of application was washed with saline. Blood flow was continuously measured until it decreased to 0. The time to 80% occlusion (blood flow reduced to 20% of the baseline blood flow) and the time to 100% occlusion (blood flow reduced to 0) were recorded. In the same animal, template bleeding time was assessed at pre-dose and 1 hour post-dose.

The effects of all three antibodies on FeCl₃-induced arterial thrombosis were investigated. Four groups of monkeys were treated with the vehicle control, h-34F8, h-19F6, or h-42A5 for 2 hours, respectively, and FeCl₃ was applied on the left femoral artery of each animal to induce thrombosis. Downstream blood flow velocity was monitored. The time to 80% and to 100% thrombotic occlusion in the vehicle control group was 14.66 ± 1.30 min and 18.50 ± 1.76 min, respectively. Pretreatment with 0.3 mg/kg of h-34F8 or h-42A5 significantly delayed the time to 80% occlusion to 59.53 ± 16.95 min and 40.80 ±7.94 min, and the time to 100% occlusion to 70.40 ± 20.76 min and 50.61 ±9.48 min, respectively, as shown in Figure 10. Prolongation of the time to 80% occlusion (26.43 ± 5.72 min) and to 100% occlusion (32.78 ± 5.09 min) was also observed in monkeys treated with h-19F6, although there was no statistically significant difference when compared to the vehicle control group as shown in Figure 10.

The effects of the antibodies on haemostasis was assessed in terms of template bleeding time. No significant difference was noted between pre-dose and 1 hour post-dose for each test article (Figure 11A, 11B, and 11C). The bleeding time change following h-34F8, h-19F6, and h-42A5 treatment was not different from that following the vehicle control treatment (Figure 11D).

The effects of the antibodies on *ex vivo* clotting times of monkey plasma were also evaluated. As expected, treatments with 0.3 mg/kg of h-34F8, h-19F6, and h-42A5 significantly prolonged APTT by 3.29 ± 0.20, 1.67 ± 0.09, and 2.87 ± 0.10 fold, respectively, while no increase in APTT was observed upon vehicle control treatment, as shown in Figure 12A. In addition, treatments with h-34F8, h-19F6, or h-42A5 had no effect on PT as shown in Figure 12B.

Thus, it was unexpectedly discovered that the antibodies disclosed herein did not have any adverse effects of prolonged bleeding while effectively inhibiting the intrinsic pathway of coagulation.

## Claims

1. An isolated anti-FXI or anti-FXla antibody that specifically binds to human FXI or FXla, wherein the antibody comprises an immunoglobulin light chain variable domain comprising CDR-L1 with SEQ ID NO: 123, CDR-L2 with SEQ ID NO: 124 and CDR-L3 with SEQ ID NO: 125 and an immunoglobulin heavy chain variable domain comprising CDR-H1 with SEQ ID NO: 126, CDR-H2 with SEQ ID NO: 127 and CDR-H3 with SEQ ID NO: 128; or a FXI- or FXla-binding fragment thereof.

2. The antibody of claim 1, wherein the antibody specifically binds to the A3 domain of the human FXI or FXla.

3. The antibody of claim 1, wherein the antibody comprises an immunoglobulin light chain variable domain with SEQ ID NO: 121.

4. The antibody of claim 1, wherein the antibody comprises an immunoglobulin heavy chain variable domain with SEQ ID NO: 122.

5. A pharmaceutical composition comprising the antibody of any one of claims 1-4.

6. The antibody of any one of claims 1-4 for use in a method for inhibiting the formation of blood clots in a subject in need thereof.

7. The antibody of any one of claims 1-4 for use in a method for treating or preventing sepsis in a subject in need thereof.

8. A method of producing the antibody of any one of claims 1-4, comprising cloning a nucleic acid encoding the antibody in an expression vector, and expressing the same in a host cell.

9. The method of claim 8, further comprising purifying the expressed antibody from the host cell.

10. The method of claim 8, wherein the host cell is a CHO cell or an HEK293T cell.

## Patentansprüche

1. Isolierter Anti-FXI- oder Anti-FXla-Antikörper, der spezifisch an humanen FXI oder FXla bindet, wobei der Antikörper eine variable Domäne einer leichten Kette eines Immunglobulins, die CDR-L1 mit SEQ-ID-NO: 123, CDR-L2 mit SEQ-ID-NO: 124 und CDR-L3 mit SEQ-ID-NO: 125 umfasst, und eine variable Domäne einer schweren Kette eines Immunglobulins, die CDR-H1 mit SEQ-ID-NO: 126, CDR-H2 mit SEQ-ID-NO: 127 und CDR-H3 mit SEQ-ID-NO: 128 umfasst, umfasst; oder ein FXI- oder FXla-Bindungsfragment davon.

2. Antikörper nach Anspruch 1, wobei der Antikörper spezifisch an die A3-Domäne des humanen FXI oder FXla bindet.

3. Antikörper nach Anspruch 1, wobei der Antikörper eine variable Domäne einer leichten Kette eines Immunglobulins mit SEQ-ID-NO: 121 umfasst.

4. Antikörper nach Anspruch 1, wobei der Antikörper eine variable Domäne einer schweren Kette eines Immunglobulins mit SEQ-ID-NO: 122 umfasst.

5. Pharmazeutische Zusammensetzung, umfassend den Antikörper nach einem der Ansprüche 1-4.

6. Antikörper nach einem der Ansprüche 1-4 zur Verwendung bei einem Verfahren zur Inhibierung der Bildung von Blutgerinnseln bei einem Subjekt, das dieses benötigt.

7. Antikörper nach einem der Ansprüche 1-4 zur Verwendung bei einem Verfahren zur Behandlung oder Prävention einer Sepsis bei einem Subjekt, das dieses benötigt.

8. Verfahren zur Herstellung des Antikörpers nach einem der Ansprüche 1-4, umfassend das Klonieren einer den Antikörper codierenden Nukleinsäure in einen Expressionsvektor und Exprimieren desselben in einer Wirtszelle.

9. Verfahren nach Anspruch 8, ferner umfassend das Reinigen des exprimierten Antikörpers aus der Wirtszelle.

10. Verfahren nach Anspruch 8, wobei die Wirtszelle eine CHO-Zelle oder eine HEK293T-Zelle ist.

## Revendications

1. Anticorps anti-FXI ou anti-FXla isolé qui se lie spécifiquement au FXI ou au FXla humain, l'anticorps comprenant un domaine variable de chaîne légère d'immunoglobuline comprenant CDR-L1 avec SEQ ID NO : 123, CDR-L2 avec SEQ ID NO : 124 et CDR-L3 avec SEQ ID NO : 125 et un domaine variable de chaîne lourde d'immunoglobuline comprenant CDR-H1 avec SEQ ID NO : 126, CDR-H2 avec SEQ ID NO : 127 et CDR-H3 avec SEQ ID NO : 128 ; ou fragment de celui-ci se liant à FXI ou FXIa.

2. Anticorps selon la revendication 1, l'anticorps se liant spécifiquement au domaine A3 du FXI ou du FXla humain.

3. Anticorps selon la revendication 1, l'anticorps comprenant un domaine variable de chaîne légère d'immunoglobuline avec SEQ ID NO : 121.

4. Anticorps selon la revendication 1, l'anticorps comprenant un domaine variable de chaîne lourde d'immunoglobuline avec SEQ ID NO : 122.

5. Composition pharmaceutique comprenant l'anticorps selon l'une quelconque des revendications 1 à 4.

6. Anticorps selon l'une quelconque des revendications 1 à 4, pour utilisation dans un procédé d'inhibition de la formation de caillots sanguins chez un sujet qui en a besoin.

7. Anticorps selon l'une quelconque des revendications 1 à 4, pour utilisation dans un procédé de traitement ou de prévention de la septicémie chez un sujet qui en a besoin.

8. Procédé de production de l'anticorps selon l'une quelconque des revendications 1 à 4, comprenant le clonage d'un acide nucléique codant pour l'anticorps dans un vecteur d'expression, et l'expression de celui-ci dans une cellule hôte.

9. Procédé selon la revendication 8, comprenant en outre la purification de l'anticorps exprimé à partir de la cellule hôte.

10. Procédé selon la revendication 8, dans lequel la cellule hôte est une cellule CHO ou une cellule HEK293T.
